(19) Europäisches Patentamt / European Patent Office / Office européen des brevets

(11) EP 4 650 350 A1

(12) EUROPEAN PATENT APPLICATION
published in accordance with Art. 153(4) EPC

(43) Date of publication:
19.11.2025 Bulletin 2025/47

(21) Application number: 24741565.6

(22) Date of filing: 11.01.2024

(51) International Patent Classification (IPC):
*C07D 303/27* (2006.01) *C08G 59/06* (2006.01)

(52) Cooperative Patent Classification (CPC):
C07D 303/27; C08G 59/06

(86) International application number:
PCT/JP2024/000435

(87) International publication number:
WO 2024/150793 (18.07.2024 Gazette 2024/29)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 12.01.2023 JP 2023002992

(71) Applicant: Mitsubishi Chemical Corporation
Tokyo 100-8251 (JP)

(72) Inventors:
- URANO, Wataru
Tokyo 100-8251 (JP)
- YOSHIMURA, Ryo
Tokyo 100-8251 (JP)

(74) Representative: TBK
Bavariaring 4-6
80336 München (DE)

# (54) POLYCRYSTALLINE FORM OF BIPHENOL DIGLYCIDYL ETHER, AND RESIN COMPOSITION CONTAINING SAID POLYCRYSTALLINE FORM AND CURED PRODUCT THEREOF

(57) Provided is a polycrystalline body of biphenol type diglycidyl ether, including an epoxy compound represented by Formula (1), and having a crystallite size of 355 Å or more and 100000 Å or less, calculated from a peak having a diffraction angle (2θ) of 8.9 to 9.3 deg in a powder X-ray diffraction pattern measured with a CuKα ray. In Formula (1), each of $R^1$ to $R^8$ is independently a hydrogen atom, an alkyl group that may have a substituent and has 1 to 12 carbon atoms, an alkoxy group that may have a substituent and has 1 to 12 carbon atoms, an aryl group that may have a substituent and has 6 to 12 carbon atoms, an alkenyl group that may have a substituent and has 2 to 12 carbon atoms, or an alkynyl group that may have a substituent and has 2 to 12 carbon atoms.

[Chem 1]

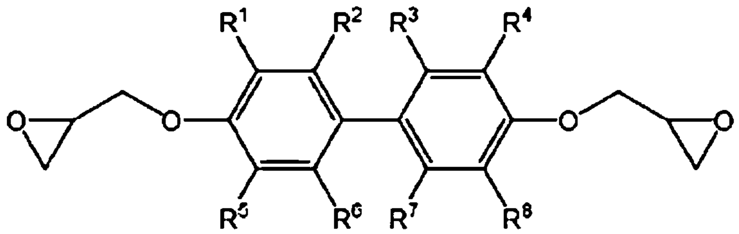

(1)

EP 4 650 350 A1

## Description

### TECHNICAL FIELD

The present invention relates to: a cured product that has high handleability industrially advantageous for a polycrystalline body of biphenol type diglycidyl ether, and is obtained by curing a polycrystalline body; and an electric and electronic component.

### BACKGROUND ART

Cured products excellent in mechanical properties, heat resistance, electrical properties, and the like have been commonly made by curing epoxy resins with various curing agents. Therefore, such epoxy resins have been utilized in the wide fields of adhesive agents, paints, electric and electronic materials, and the like. Tetramethylbiphenol type epoxy resins have been frequently used because of enabling high-value-added sealing materials to be provided, particularly in the applications of semiconductor sealing materials, among the fields of electric and electronic materials.

As recent trends in technology for producing semiconductor sealing materials, the following (1) and (2) have been demanded.

(1) High Productivity Caused by Dry Blending in Advance in Production of Semiconductor Sealing Material

A solid sealing material is widely used as a semiconductor sealing material from the viewpoint of moldability and storage properties. A method of homogenizing raw material components by dry blending in advance is adopted in order to enhance production efficiency in production of the semiconductor sealing material and to enhance the homogeneity of the sealing material. Solid and powdery materials are preferred as an epoxy resin, a curing agent, and an inorganic filler used as the raw materials.

(2) Decrease in Melt Viscosity by Crystalline Raw Material

Downsizing of a semiconductor causes thinning of a wire through which IC chips are connected to each other. To prevent wire flow, high fluidity is demanded in a semiconductor sealing material that protects the wire. Among solid epoxy resins which are raw materials, a crystalline epoxy resin having a lower melt viscosity is preferred.

Patent Literatures 1 and 2 describe that a tetramethylbiphenol type epoxy resin was produced by reaction between 4,4'-bishydroxy-3,3',5,5'-tetramethylbiphenyl and epichlorohydrin.

### PRIOR ART DOCUMENTS

### PATENT DOCUMENTS

Patent Document 1: Japanese Unexamined Patent Application Publication No. S58-039677
Patent Document 2: Japanese Unexamined Patent Application Publication No. 2004-315832

### SUMMARY OF INVENTION

### TECHNICAL PROBLEM

An epoxy resin, a curing agent, and an inorganic filler which are raw materials are premixed by dry blending, and then melt-kneaded by a kneader or a heated roll, to produce a semiconductor sealing material. Amorphous epoxy resins have been conventionally used as epoxy resins. However, such an amorphous epoxy resin has problems in that the amorphous epoxy resin has a low softening point, and is inhibited from being homogenized due to generation of a lump by adhesion between solid raw materials in the case of preliminarily kneading the amorphous epoxy resin by dry blending, and in that the amorphous epoxy resin has low productivity in the case of melt-kneading the amorphous epoxy resin because the amorphous epoxy resin has a high melt viscosity. Thus, the industrialization of a powdery crystalline epoxy resin has been desired for simultaneously solving the two problems described above. In such a crystalline epoxy resin, raw materials are homogeneously mixed in the case of kneading the crystalline epoxy resin in a short time, and improvement in productivity is expected because the crystalline epoxy resin has a sufficiently high melting point to prevent a lump from being generated in the case of dry blending, and has a low melt viscosity.

Moreover, downsizing of each constitutional unit has been allowed to proceed in order to improve the processing speeds of semiconductors. From this viewpoint, thinning of wires through which IC chips have been connected to each other has been allowed to proceed, and semiconductor sealing materials that protect the IC chips have required low viscosity characteristics in order to prevent wire flow. To satisfy this objective, such a crystalline epoxy resin is expected to be a preferred raw material due to a low viscosity.

An epoxy resin according to Patent Literature 1 has had the insufficiently satisfiable handleability of the dry blend of a solid raw material, important for productivity, in a semiconductor sealing material production process of efficiently producing a powdery epoxy resin, used as a crystalline raw material for a semiconductor sealing material, in an industrial scale, and making a cured product by using the epoxy resin.

A crystallization step is commonly susceptible to the influences of an air interface and the surface material and shape of a package in a case in which the crystallized product of an epoxy resin is produced on an industrial scale. A method of producing a crystallized product according to Patent Literature 2 is more highly susceptible to the influences. Therefore, an influence on a process of generating a crystallized product in a long period, important for homogeneously crystallizing a portion up to the interior of the crystallized product, necessary for grinding the crystallized product, is unclear. Thus, it is not clear whether a crystalline body with homogeneous crystal properties, which can endure grinding on an industrial scale, is obtained.

Thus, an objective of the present invention is to provide: a polycrystalline body of biphenol type diglycidyl ether, which is excellent in grindability and fluidity, and can therefore improve productivity in production of an epoxy resin, a semiconductor sealing material, and the like; a resin composition including the polycrystalline body; a cured product including a resin composition; and an electric and electronic component.

SOLUTION TO PROBLEM

As a result of intensive examination for solving the problems described above, the present inventors found that a polycrystalline body of biphenol type diglycidyl ether, obtained by adjusting a crystallite size, calculated from a peak detected by powder X-ray diffraction, to a specific value can solve the above-described problems, and the present invention was thus accomplished.

In other words, the gist of the present invention is in the following [1] to [10].

[1] A polycrystalline body of biphenol type diglycidyl ether, including an epoxy compound represented by the following Formula (1), and having a crystallite size of 355 Å or more and 100000 Å or less, calculated from a peak having a diffraction angle (2θ) of 8.9 to 9.3 deg in a powder X-ray diffraction pattern measured with a CuKα ray,

[Chem 1]

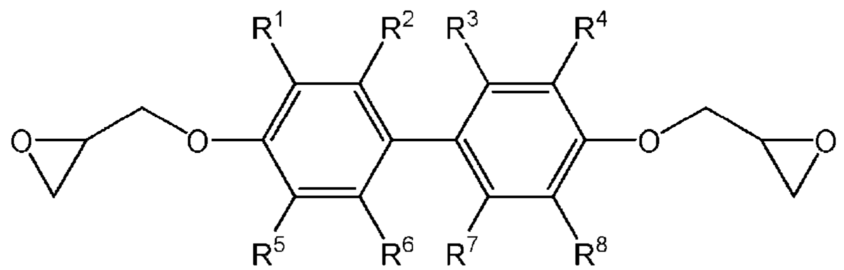

(1)

(in Formula (1) described above, each of $R^1$ to $R^8$ is independently a hydrogen atom, an alkyl group that may have a substituent and has 1 to 12 carbon atoms, an alkoxy group that may have a substituent and has 1 to 12 carbon atoms, an aryl group that may have a substituent and has 6 to 12 carbon atoms, an alkenyl group that may have a substituent and has 2 to 12 carbon atoms, or an alkynyl group that may have a substituent and has 2 to 12 carbon atoms).

[2] The polycrystalline body of the biphenol type diglycidyl ether according to [1], wherein the polycrystalline body has an average particle diameter of 0.5 to 10000 μm.

[3] The polycrystalline body of the biphenol type diglycidyl ether according to [1] or [2], wherein the polycrystalline body is a ground product.

[4] The polycrystalline body of the biphenol type diglycidyl ether according to any of [1] to [3], wherein the polycrystalline body has a melting point of 80°C or more.

[5] The polycrystalline body of the biphenol type diglycidyl ether according to any of [1] to [4], wherein the polycrystalline body has a crystallite size of 294 Å or more and 100000 Å or less, calculated from a peak having a diffraction angle (2θ) of 15.1 to 15.5 deg in the powder X-ray diffraction pattern measured with the CuKα ray.

[6] The polycrystalline body of the biphenol type diglycidyl ether according to any of [1] to [5], wherein the polycrystalline body further has diffraction peaks at diffraction angles (2θ) of 15.1 to 15.5 deg, 19.4 to 19.8 deg, and 24.9 to 25.3 deg in the powder X-ray diffraction pattern measured with the CuKα ray.

[7] The polycrystalline body of the biphenol type diglycidyl ether according to any of [1] to [6], wherein the polycrystalline body further includes an epoxy compound represented by the following Formula (2):

[Chem 2]

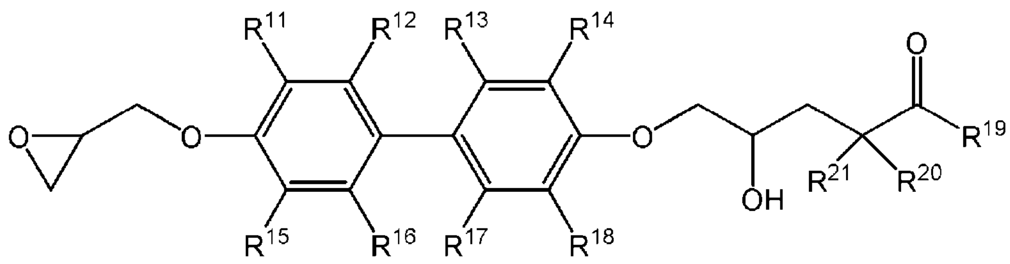

(2)

(in Formula (2), each of $R^{11}$ to $R^{18}$ is independently a hydrogen atom, an alkyl group that may have a substituent and has 1 to 12 carbon atoms, an alkoxy group that may have a substituent and has 1 to 12 carbon atoms, an aryl group that may have a substituent and has 6 to 12 carbon atoms, an alkenyl group that may have a substituent and has 2 to 12 carbon atoms, or an alkynyl group that may have a substituent and has 2 to 12 carbon atoms; each of $R^{19}$ to $R^{21}$ is independently a hydrogen atom, an alkyl group that may have a substituent and has 1 to 12 carbon atoms, or an aryl group that may have a substituent and may have a substituent having 6 to 12 carbon atoms; and $R^{19}$ and $R^{20}$ may be bound to each other to form a cyclic structure having 1 to 12 carbon atoms).

[8] A resin composition including: the polycrystalline body of the biphenol type diglycidyl ether according to any of [1] to [7]; and a curing agent, wherein the resin composition includes 0.01 to 1000 parts by mass of the curing agent with respect to 100 parts by mass of a total epoxy resin component as a solid content.

[9] The resin composition according to [8], wherein the curing agent is at least one selected from a group consisting of phenolic curing agents, amine-based curing agents, acid anhydride-based curing agents, and amide-based curing agents.

[10] A cured product of the resin composition according to [8] or [9].

[11] An electric and electronic component including a resin composition including the polycrystalline body of the biphenol type diglycidyl ether according to any of [1] to [7].

[12] An electric and electronic component including a cured product of a resin composition including the polycrystalline body of the biphenol type diglycidyl ether according to any of [1] to [7].

## ADVANTAGEOUS EFFECTS OF INVENTION

In accordance with the present invention, a polycrystalline body of biphenol type diglycidyl ether, which is excellent in grindability and fluidity, can be obtained. The polycrystalline body of the biphenol type diglycidyl ether is excellent in grindability in an industrial scale, has a melting point in a desirable range from the viewpoint of production of a semiconductor, and is also excellent in fluidity. Therefore, the polycrystalline body is expected to have high industrial handleability particularly in the case of producing a semiconductor.

## DESCRIPTION OF EMBODIMENTS

Embodiment of the present invention are described in detail below. The following description is an example of the embodiments of the present invention. The present invention is not limited to the following description unless deviating from the gist of the present invention.

Herein, an expression of "x to y" is used to include x and y, which are numerical values or physical property values.

Epoxy resins in the present embodiment include an epoxy resin including a repeated structure and an epoxy resin including a monomolecular structure. Each of the epoxy compounds may be expressed as and sold under "epoxy resin" or "epoxy resin composition" in the industry of epoxy resins. In the industry of the epoxy resins, a mixture further including an epoxy resin different from the epoxy resins in the present embodiment may be expressed as "epoxy resin composition", or may be simply referred to as "epoxy resin".

[Polycrystalline Body of Biphenol Type Diglycidyl Ether]

A polycrystalline body of biphenol type diglycidyl ether according to one embodiment of the present invention (hereinafter, may be simply referred to as "polycrystalline body of biphenol type diglycidyl ether") includes an epoxy compound represented by the following Formula (1) (hereinafter, may be referred to as "epoxy compound (1)" or "epoxy resin (1)"), and has a crystallite size of 355 Å or more and 100000 Å or less, calculated from a peak having a diffraction angle (2θ) of 8.9 to 9.3 deg in a powder X-ray diffraction pattern measured with a CuKα ray.

[Chem 3]

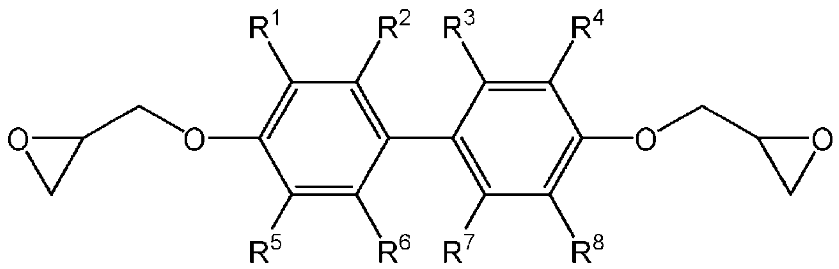

(1)

Each of $R^1$ to $R^8$ is independently a hydrogen atom, an alkyl group having 1 to 12 carbon atoms, an alkoxy group having 1 to 12 carbon atoms, an aryl group having 6 to 12 carbon atoms, an alkenyl group having 2 to 12 carbon atoms, or an alkynyl group having 2 to 12 carbon atoms, and each of the alkyl group, the alkoxy group, the aryl group, the alkenyl group, and the alkynyl group may include a substituent, or need not include any substituent.

Examples of the alkyl group having 1 to 12 carbon atoms include a methyl group, an ethyl group, an n-propyl group, an isopropyl group, an n-butyl group, a sec-butyl group, a tert-butyl group, an n-pentyl group, an isopentyl group, a neopentyl group, a tert-pentyl group, a cyclopentyl group, an n-hexyl group, an isohexyl group, a cyclohexyl group, an n-heptyl group, a cycloheptyl group, a methylcyclohexyl group, an n-octyl group, a cyclooctyl group, an n-nonyl group, a 3,3,5-trimethylcyclohexyl group, an n-decyl group, a cyclodecyl group, an n-undecyl group, an n-dodecyl group, a cyclododecyl group, a benzyl group, a methylbenzyl group, a dimethylbenzyl group, a trimethylbenzyl group, a naphthylmethyl group, a phenethyl group, or a 2-phenylisopropyl group.

Examples of the alkoxy group include a methoxy group, an ethoxy group, an n-propoxy group, an isopropoxy group, an n-butoxy group, a sec-butoxy group, a tert-butoxy group, an n-pentoxy group, an isopentoxy group, a neopentoxy group, a tert-pentoxy group, a cyclopentoxy group, an n-hexyloxy group, an isohexyloxy group, a cyclohexyloxy group, an n-heptoxy group, a cycloheptoxy group, a methylcyclohexyloxy group, an n-octyloxy group, a cyclooctyloxy group, an n-nonyloxy group, a 3,3,5-trimethylcyclohexyloxy group, an n-decyloxy group, a cyclodecyloxy group, an n-undecyloxy group, an n-dodecyloxy group, a cyclododecyloxy group, a benzyloxy group, a methylbenzyloxy group, a dimethylbenzyloxy group, a trimethylbenzyloxy group, a naphthylmethoxy group, a phenethyloxy group, or a 2-phenylisopropoxy group.

Examples of the alkenyl group include a vinyl group, a 1-propenyl group, a 2-propenyl group, a 1-methylvinyl group, a 1-butenyl group, a 2-butenyl group, a 3-butenyl group, a 1,3-butadienyl group, a cyclohexenyl group, a cyclohexadienyl group, a cinnamyl group, or a naphthylvinyl group.

Examples of the alkynyl group include an ethynyl group, a 1-propynyl group, a 2-propynyl group, a 1-butynyl group, a 2-butynyl group, a 3-butynyl group, a 1,3-butanedienyl group, a phenylethynyl group, or a naphthylethynyl group.

Examples of the aryl group include a phenyl group, an o-tolyl group, an m-tolyl group, a p-tolyl group, an ethylphenyl group, a styryl group, a xylyl group, an n-propylphenyl group, an isopropylphenyl group, a mesityl group, an ethynylphenyl group, a naphthyl group, or a vinylnaphthyl group.

Each of $R^1$ to $R^8$ is preferably independently a hydrogen atom, an alkyl group that may have a substituent and has 1 to 12 carbon atoms, or an aryl group that may have a substituent and has 6 to 12 carbon atoms, more preferably independently a hydrogen atom or an alkyl group that may have a substituent and has 1 to 12 carbon atoms, still more preferably a hydrogen atom or an alkyl group that may have a substituent and 1 to 6 carbon atoms, and particularly preferably a hydrogen atom or an alkyl group that may have a substituent and has 1 to 3 carbon atoms.

In $R^1$ to $R^8$, the substituent that may be included in the alkyl group having 1 to 12 carbon atoms, the alkoxy group having 1 to 12 carbon atoms, the aryl group having 6 to 12 carbon atoms, the alkenyl group having 2 to 12 carbon atoms, or the alkynyl group having 2 to 12 carbon atoms is not particularly limited, and examples thereof include a cyano group, an amino group, a carboxyl group, an ester group, an alkylcarbonyl group, an acetyl group, a sulfonyl group, a silyl group, a boryl group, a thio group, or a seleno group. From the viewpoint of reducing the water absorption percentage of a cured product, the substituent is preferably an ester group, an alkylcarbonyl group, or a silyl group.

The polycrystalline body of the biphenol type diglycidyl ether may be a ground product. Herein, the ground product is a substance obtained by grinding. Specifically, the ground product is preferably a particulate substance having an average particle diameter of 5 cm or less. The conditions of the parameters of the size of the polycrystalline body are described below, and the conditions are particularly preferred in a case in which the polycrystalline body is a ground product.

The average particle diameter of the polycrystalline body of the biphenol type diglycidyl ether is not particularly limited, and is typically 0.5 μm or more, and preferably 1 μm or more. The lower limit value of the average particle diameter is more preferably 5 μm or more, and still more preferably 20 μm or more. In a case in which the average particle diameter is in a range of not less than the lower limit value, it is easy to prevent a fine powder from adhering to an apparatus such as a grinding apparatus, and to prevent powder dust from being stirred up, in treatment such as grinding treatment.

The average particle diameter of the polycrystalline body of the biphenol type diglycidyl ether is preferably 10000 μm or less. The upper limit value of the average particle diameter is more preferably 5000 μm or less, and still more preferably 2000 μm or less. In a case in which the average particle diameter is in a range of not more than the upper limit value of the range described above, the polycrystalline body is easily melted in a short time in melt-kneading in the case of producing a semiconductor sealing material, and is easily homogenized in the case of mixing the polycrystalline body with another epoxy resin, a curing agent, an inorganic filler, or the like.

The D10 value of the polycrystalline body of the biphenol type diglycidyl ether is not particularly limited, and is preferably 0.1 μm or more. The lower limit value of the D10 value is more preferably 1 μm or more, and still more preferably 10 μm or more. In a case in which the D10 value is not less than the lower limit value described above, it is easy to prevent a fine powder from adhering to an apparatus such as a grinding apparatus, and to prevent powder dust from being stirred up, in treatment such as grinding treatment.

The D10 value of the polycrystalline body of the biphenol type diglycidyl ether is preferably 1000 μm or less. The upper limit value of the D10 value is more preferably 800 μm or less, and still more preferably 500 μm or less. In a case in which the D10 value is in a range of not more than the upper limit value of the range described above, the polycrystalline body is easily melted in a short time in melt-kneading in the case of producing a semiconductor sealing material, and is easily homogenized in the case of mixing the polycrystalline body with another epoxy resin, a curing agent, an inorganic filler, or the like.

The D50 value of the polycrystalline body of the biphenol type diglycidyl ether is not particularly limited, and is preferably 1 μm or more. The lower limit value of the D50 value is more preferably 5 μm or more, and still more preferably 20 μm or more. In a case in which the D50 value is in a range of not less than the lower limit value described above, it is easy to prevent a fine powder from adhering to an apparatus such as a grinding apparatus, and to prevent powder dust from being stirred up, in treatment such as grinding treatment.

The D50 value of the polycrystalline body of the biphenol type diglycidyl ether is preferably 10000 μm or less. The upper limit value of the D50 value is more preferably 5000 μm or less, and still more preferably 2000 μm or less. In a case in which the D50 value is in a range of not more than the upper limit value of the range described above, the polycrystalline body is easily melted in a short time in melt-kneading in the case of producing a semiconductor sealing material, and is easily homogenized in the case of mixing the polycrystalline body with another epoxy resin, a curing agent, an inorganic filler, or the like.

The D90 value of the polycrystalline body of the biphenol type diglycidyl ether is not particularly limited, and is preferably 5 μm or more. The lower limit value of the D90 value is more preferably 10 μm or more, and still more preferably 30 μm or more. In a case in which the D90 value is in a range of not less than the lower limit value described above, it is easy to prevent a fine powder from adhering to an apparatus such as a grinding apparatus, and to prevent powder dust from being stirred up, in treatment such as grinding treatment.

The D90 value of the polycrystalline body of the biphenol type diglycidyl ether is preferably 20000 μm or less. The upper limit value of the D90 value is more preferably 10000 μm or less, and still more preferably 3000 μm or less. In a case in which the D90 value is in a range of not more than the upper limit value of the range described above, the polycrystalline body is easily melted in a short time in melt-kneading in the case of producing a semiconductor sealing material, and is easily homogenized in the case of mixing the polycrystalline body with another epoxy resin, a curing agent, an inorganic filler, or the like.

The average particle diameter, D10 value, D50 value, and D90 value of the ground product of the polycrystalline body described above can be measured by, for example, a laser scattering method or a sieve method.

The melting point of the polycrystalline body of the biphenol type diglycidyl ether is not particularly limited, and is preferably 80°C or more. The lower limit value of the melting point is more preferably 85°C or more, and still more preferably 90°C or more. In a case in which the melting point is in a range of not less than the lower limit value described above, melting caused by heat generated by, for example, treatment of grinding a polycrystalline body, and adhesion in an apparatus are inhibited.

The melting point of the polycrystalline body of the biphenol type diglycidyl ether is preferably 150°C or less. The upper limit value of the melting point is more preferably 140°C or less, and still more preferably 130°C or less. In a case in

which the melting point is in a range of not more than the upper limit value of the range described above, the polycrystalline body is easily melted in a short time in melt-kneading in the case of producing a semiconductor sealing material, and is easily homogenized in the case of mixing the polycrystalline body with another epoxy resin, a curing agent, an inorganic filler, or the like.

In a case in which the polycrystalline body of the biphenol type diglycidyl ether has two or more melting points, any one of the plurality of melting points preferably satisfies the range described above, and all the melting points more preferably satisfy the range described above.

In the polycrystalline body of the biphenol type diglycidyl ether, the content of the epoxy resin (1) is not particularly limited, and is preferably 70.0 to 99.9% by mass, more preferably 75.0 to 98.0% by mass, and still more preferably 80.0 to 95.0% by mass with respect to 100% by mass of the polycrystalline body of the biphenol type diglycidyl ether. In a case in which the content is not less than the lower limit value of the range described above, it is easy to obtain a polycrystalline body having a large crystallite size, suitable for treatment such as grinding. In a case in which the content is not more than the upper limit value of the range described above, the melting point is decreased, and raw materials for a semiconductor sealing material can be mixed in a short time.

The content of the epoxy resin (1) described above can be evaluated by a method described in Examples described later.

The polycrystalline body of the biphenol type diglycidyl ether has a diffraction peak at a diffraction angle (2θ) of 8.9 to 9.3 deg, preferably has a diffraction peak at a diffraction angle (2θ) in at least one range selected from the group consisting of 15.1 to 15.5 deg, 19.4 to 19.8 deg, and 24.9 to 25.3 deg, and more preferably has diffraction peaks at diffraction angles (2θ) in all the ranges thereof, in a powder X-ray diffraction pattern measured with a CuKα ray.

The crystallite size of the polycrystalline body, calculated from the peak having a diffraction angle (2θ) of 8.9 to 9.3 deg in the powder X-ray diffraction pattern measured with a CuKα ray, is 355 Å or more and 100000 Å or less. The crystallite size is preferably 375 Å or more and 10000 Å or less, and more preferably 400 Å or more and 1000 Å or less. In a case in which the crystallite size is not less than the lower limit value described above, adhesion of the polycrystalline body in treatment such as grinding is inhibited, and in a case in which the crystallite size is not more than the upper limit value of the range described above, the crystallite size is small, the particle diameter after the treatment is decreased, and the polycrystalline body is melted in heating in a short time, that is, excellent fluidity is obtained. A diffraction angle of 8.9 to 9.3 deg is in a range preferable for calculating a crystallite size because the influence of another peak is inhibited in comparison with 15.1 to 15.5 deg, 19.4 to 19.8 deg, and 24.9 to 25.3 deg.

The crystallite size of the polycrystalline body, calculated from the peak having a diffraction angle (2θ) of 15.1 to 15.5 deg in the powder X-ray diffraction pattern measured with a CuKα ray, is preferably 294 Å or more and 100000 Å or less, more preferably 300 Å or more and 10000 Å or less, and still more preferably 306 Å or more and 1000 Å or less. In a case in which the crystallite size is not less than the lower limit value described above, adhesion of the polycrystalline body in treatment such as grinding is inhibited, and in a case in which the crystallite size is not more than the upper limit value of the range described above, the crystallite size is small, the particle diameter after the treatment is decreased, and the polycrystalline body is melted in heating in a short time.

The crystallite size of the polycrystalline body, calculated from the peak having a diffraction angle (2θ) of 19.4 to 19.8 deg in the powder X-ray diffraction pattern measured with a CuKα ray, is preferably 260 Å or more and 100000 Å or less, more preferably 270 Å or more and 10000 Å or less, and still more preferably 280 Å or more and 1000 Å or less. In a case in which the crystallite size is not less than the lower limit value described above, adhesion of the polycrystalline body in treatment such as grinding is inhibited, and in a case in which the crystallite size is not more than the upper limit value of the range described above, the crystallite size is small, the particle diameter after the treatment is decreased, and the polycrystalline body is melted in heating in a short time.

The crystallite size of the polycrystalline body, calculated from the peak having a diffraction angle (2θ) of 24.9 to 25.3 deg in the powder X-ray diffraction pattern measured with a CuKα ray, is preferably 255 Å or more and 100000 Å or less, more preferably 260 Å or more and 10000 Å or less, and still more preferably 265 Å or more and 1000 Å or less. In a case in which the crystallite size is not less than the lower limit value described above, adhesion of the polycrystalline body in treatment such as grinding is inhibited, and in a case in which the crystallite size is not more than the upper limit value of the range described above, the crystallite size is small, the particle diameter after the treatment is decreased, and the polycrystalline body is melted in heating in a short time.

The crystallite size of the polycrystalline body described above can be evaluated by the method described in Examples described later.

The polycrystalline body of the biphenol type diglycidyl ether preferably includes an epoxy compound represented by the following Formula (2) (hereinafter, may be referred to as "epoxy compound (2)" or "epoxy resin (2)") from the viewpoint of decreasing a melting point and shortening fusion time.

[Chem 4]

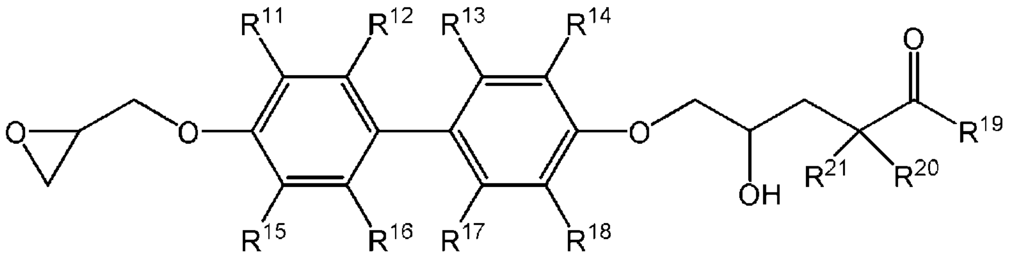

(2)

In Formula (2) described above, $R^{11}$ to $R^{18}$ are synonymous with $R^1$ to $R^8$ in Formula (1) described above, respectively.

Each of $R^{19}$ to $R^{21}$ is independently a hydrogen atom, an alkyl group having 1 to 12 carbon atoms, or an aryl group having 6 to 12 carbon atoms, and the alkyl group and the aryl group may have a substituent. Moreover, $R^{19}$ and $R^{20}$ may be bound to each other to form a cyclic structure having 1 to 12 carbon atoms.

Examples of the alkyl group having 1 to 12 carbon atoms include a methyl group, an ethyl group, an n-propyl group, an isopropyl group, an n-butyl group, a sec-butyl group, an isobutyl group, a tert-butyl group, an n-pentyl group, an isopentyl group, a neopentyl group, a tert-pentyl group, a cyclopentyl group, an n-hexyl group, an isohexyl group, a cyclohexyl group, an n-heptyl group, a cycloheptyl group, a methylcyclohexyl group, an n-octyl group, a cyclooctyl group, an n-nonyl group, a 3,3,5-trimethylcyclohexyl group, an n-decyl group, a cyclodecyl group, an n-undecyl group, an n-dodecyl group, a cyclododecyl group, a benzyl group, a methylbenzyl group, a dimethylbenzyl group, a trimethylbenzyl group, a naphthylmethyl group, a phenethyl group, or a 2-phenylisopropyl group.

Examples of the aryl group having 6 to 12 carbon atoms include a phenyl group, an o-tolyl group, an m-tolyl group, a p-tolyl group, an ethylphenyl group, a styryl group, a xylyl group, an n-propylphenyl group, an isopropylphenyl group, a mesityl group, an ethynylphenyl group, a naphthyl group, or a vinylnaphthyl group.

In a case in which $R^{19}$ and $R^{20}$ are bound to each other to form a cyclic structure, it is preferable to form a cyclic ketone structure having 3 to 12 carbon atoms. Examples of the cyclic structure include cyclopropanone, cyclobutanone, cyclohexanone, cycloheptanone, cyclooctanone, cyclononanone, cyclodecanone, or cyclododecanone.

Each of $R^{19}$ to $R^{21}$ is preferably independently a hydrogen atom or an alkyl group that may have a substituent and has 1 to 12 carbon atoms, and more preferably independently a hydrogen atom or an alkyl group that may have a substituent and has 1 to 6 carbon atoms. Most preferably, $R_9$ is an isobutyl group, and $R^{20}$ and $R^{21}$ are hydrogen atoms.

In $R^{19}$ to $R^{21}$, the substituent that may be included in the alkyl group having 1 to 12 carbon atoms, the alkoxy group having 1 to 12 carbon atoms, the aryl group having 6 to 12 carbon atoms, the alkenyl group having 2 to 12 carbon atoms, or the alkynyl group having 2 to 12 carbon atoms is not particularly limited, and examples thereof include a cyano group, an amino group, a carboxyl group, an ester group, an alkylcarbonyl group, an acetyl group, a sulfonyl group, a silyl group, a boryl group, a thio group, or a seleno group. From the viewpoint of reducing the water absorption percentage of a cured product, the substituent is preferably an ester group, an alkylcarbonyl group, or a silyl group.

In the polycrystalline body of the biphenol type diglycidyl ether, the content of the epoxy resin (2) is not particularly limited, and is preferably 0 to 10.0% by mass (0% by mass means less than the detection limit), more preferably 0.01 to 10.0% by mass, still more preferably 0.1 to 5.0% by mass, and particularly preferably 0.5 to 3.0% by mass with respect to 100% by mass of the polycrystalline body of the biphenol type diglycidyl ether. In a case in which the content is not less than the lower limit value of the range described above, the polycrystalline body of the biphenol type diglycidyl ether, excellent in solvent solubility, is obtained. In a case in which the content is not more than the upper limit value of the range described above, the polycrystalline body of the biphenol type diglycidyl ether can be obtained in a short time.

The content of the epoxy resin (2) described above can be evaluated by the method described in Examples described later.

The polycrystalline body of the biphenol type diglycidyl ether may include, or need not include a component (another component) other than the epoxy resin (1) and epoxy resin (2) described above, within the range in which the effects of the present invention can be obtained. Examples of the other component include an epoxy resin (another epoxy resin) other than the epoxy resin (1) and epoxy resin (2) described above.

Examples of the other epoxy resin include bisphenol A type epoxy resins, trisphenol methane type epoxy resins, anthracene type epoxy resins, phenol-modified xylene resin type epoxy resins, bisphenol cyclododecyl type epoxy resins, bisphenol diisopropylidene resorcin type epoxy resins, bisphenol F type epoxy resins, bisphenol C type epoxy resins, bisphenol AD type epoxy resins, hydroquinone type epoxy resins, methylhydroquinone type epoxy resins, dibutylhydroquinone type epoxy resins, resorcin type epoxy resins, methylresorcin type epoxy resins, biphenol type epoxy resins, tetramethylbiphenol type epoxy resins other than the epoxy resins (1) and (2) described above, tetramethyl bisphenol F type epoxy resins, dihydroxydiphenyl ether type epoxy resins, epoxy resins derived from thiodiphenols, dihydroxynaphthalene type epoxy resins, dihydroxyanthracene type epoxy resins, dihydroxydihydroanthracene type epoxy resins, dicyclopentadiene type epoxy resins, epoxy resin derived from dihydroxystilbene, phenol novolac type epoxy resins,

cresol novolac type epoxy resins, bisphenol A novolac type epoxy resins, naphthol novolac type epoxy resins, phenol aralkyl type epoxy resins, naphthol aralkyl type epoxy resins, biphenyl aralkyl type epoxy resins, terpene phenol type epoxy resins, dicyclopentadiene phenol type epoxy resins, epoxy resins derived from condensates of phenolhydroxybenzaldehyde, epoxy resins derived from condensates of phenol-crotonaldehyde, epoxy resins derived from condensates of phenol-glyoxal, epoxy resins derived from cocondensated resins of heavy oils or pitches, phenols, and formaldehydes, epoxy resins derived from diaminodiphenyl methane, epoxy resins derived from aminophenol, epoxy resins derived from xylenediamine, epoxy resins derived from methyl hexahydrophthalic acid, and epoxy resins derived from dimer acids.

The total content of the other component in the polycrystalline body of the biphenol type diglycidyl ether is preferably 50% by mass or less, more preferably 30% by mass or less, and still more preferably 15% by mass or less. It is not necessary to set the lower limit of the total content. The total content may be 0% by mass (less than the detection limit), may be 1% by mass or more, and may be 5% by mass or more.

The melt viscosity of the polycrystalline body of the biphenol type diglycidyl ether at 150°C is preferably 1.0 poise or less, more preferably 0.5 poise or less, and still more preferably 0.3 poise or less. In a case in which the melt viscosity is in the range described above, the polycrystalline body of the biphenol type diglycidyl ether, resulting in shorter time for which raw materials are mixed in production of a semiconductor sealing material, can be obtained. It is not particularly necessary to set the lower limit of the melt viscosity. The lower limit is typically 0.01 poise or more, may be 0.02 poise or more, or may be 0.05 poise or more.

The melt viscosity described above can be evaluated by the method described in Examples described later.

The epoxy equivalent of the polycrystalline body of the biphenol type diglycidyl ether is not particularly limited. The epoxy equivalent is preferably 170 g/eq or more, more preferably 174 g/eq or more, and still more preferably 177 g/eq or more. Moreover, the epoxy equivalent is preferably 230 g/eq or less, more preferably 220 g/eq or less, and still more preferably 215 g/eq or less.

In a case in which the epoxy equivalent is not less than the lower limit of the range described above, the elastic modulus of a cured product is decreased to achieve excellent crack resistance. In a case in which the epoxy equivalent is not more than the upper limit of the range described above, Tg of the cured product is high, and heat resistance is excellent.

The epoxy equivalent is defined as "mass of epoxy resin including 1 equivalent of epoxy group", and can be measured according to JIS K7236.

[Method of Producing Polycrystalline Body of Biphenol Type Diglycidyl Ether]

A method of producing a polycrystalline body of biphenol type diglycidyl ether is not particularly limited. For example, the polycrystalline body can be produced by any of the following methods (hereinafter, production methods according to the following (1) to (3) may be referred to as "production method (1)", "production method (2)", and "production method (3)", respectively) after production of a tetramethyl biphenol type epoxy resin (hereinafter, may be referred to as "crude epoxy resin").

(1) The crude epoxy resin is subjected to crystallization treatment under desired crystallization conditions.
(2) The crude epoxy resin is recrystallized and then subjected to crystallization treatment under desired crystallization conditions.
(3) The crude epoxy resin is reacted with an alkali metal hydroxide, and then subjected to crystallization treatment under desired crystallization conditions.

The method of producing a polycrystalline body of biphenol type diglycidyl ether after the production of the crude epoxy resin is not particularly limited, and is preferably the production method (1) or the production method (2), and more preferably the production method (1), from the viewpoint of production efficiency in industrial production.

[Production of Crude Epoxy Resin]

A method of producing the crude epoxy resin is not particularly limited, and examples thereof include a production method by a one-step method described below.

<Method of Producing Crude Epoxy Resin by One-Step Method>

In the method of producing a crude epoxy resin by a one-step method, a crude epoxy resin as a raw material for a polycrystalline body of biphenol type diglycidyl ether is produced by reacting a biphenol compound represented by the following Formula (3) (hereinafter, may be referred to as "biphenol (3)") with an epihalohydrin.

[Chem 5]

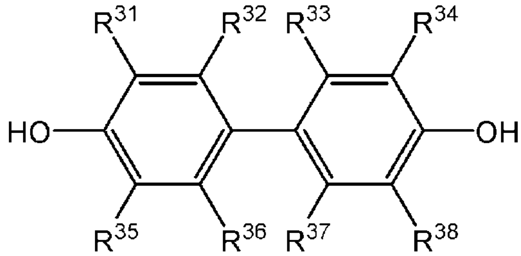

(3)

$R^{31}$ to $R^{38}$ in Formula (3) are synonymous with $R^1$ to $R^8$ in Formula (1) described above, respectively.

Examples of the biphenol (3) include biphenol, tetramethylbiphenol, 4,4'-dihydroxy-2,2',3,3',5,5'-hexamethylbiphenol, 4,4'-dihydroxy-3,3',5,5'-tetraisopropylbiphenol, and 2,2',6,6'-tetra-tert-butyl-4,4'-dihydroxybiphenol. The biphenol (3) is preferably biphenol or tetramethylbiphenol, and particularly preferably tetramethylbiphenol.

In the case of producing the crude epoxy resin by the one-step method, at least the biphenol (3) and the epihalohydrin are used as raw materials, and a multivalent hydroxy compound (hereinafter, may be referred to as "other multivalent hydroxy compound") other than the biphenol (3) may also be used together.

However, the rate of the biphenol (3) is preferably 80% by mol or more, more preferably 90% by mol or more, and still more preferably 95% by mol or more with respect to the total amount of the multivalent hydroxy compound used as a raw material from the viewpoint of enhancing the effects of the present invention. The upper limit thereof is 100% by mol. The upper limit is particularly preferably 100% by mol, and may be 100% by mol or less, less than 100% by mol, 99.9% by mol or less, 99% by mol or less, or 98% by mol or less. Herein, "multivalent hydroxy compound" is a generic name for divalent and higher-valent phenol compounds and divalent and higher valent alcohols.

Examples of such other multivalent hydroxy compounds include: various phenol resins such as: various polyphenols (excluding the biphenol (3)) such as bisphenol A, bisphenol F, bisphenol S, bisphenol AD, bisphenol AF, hydroquinone, resorcin, methylresorcin, biphenol, dihydroxynaphthalene, dihydroxydiphenyl ether, thiodiphenol, phenol novolac resin, cresol novolac resin, phenol aralkyl resin, biphenyl aralkyl resin, naphthol aralkyl resin, terpene phenol resin, dicyclopentadiene phenol resin, bisphenol A novolac resin, naphthol novolac resin, brominated bisphenol A, and brominated phenol novolac resin; polyphenol resins obtained by condensation reaction of various phenols with various aldehydes such as benzaldehyde, hydroxybenzaldehyde, crotonaldehyde, and glyoxal; polyphenol resins obtained by condensation reaction of xylene resin with phenols; and cocondensated resins of heavy oils or pitches, phenols, and formaldehydes; chain aliphatic diols such as ethylene glycol, trimethylene glycol, propylene glycol, 1,3-butanediol, 1,4-butanediol, 1,3-pentanediol, 1,4-pentanediol, 1,5-pentanediol, and 1,6-hexanediol; cyclic aliphatic diols such as cyclohexanediol and cyclodecanediol; and polyalkylene ether glycols such as polyethylene ether glycol, polyoxytrimethylene ether glycol, and polypropylene ether glycol.

Preferred examples thereof include phenol novolac resin, phenol aralkyl resin, polyphenol resins obtained by condensation reaction of phenols with hydroxybenzaldehyde, biphenyl aralkyl resin, naphthol aralkyl resin, chain aliphatic diols such as ethylene glycol, trimethylene glycol, propylene glycol, 1,3-butanediol, 1,4-butanediol, 1,3-pentanediol, 1,4-pentanediol, 1,5-pentanediol, and 1,6-hexanediol, cyclic aliphatic diols such as cyclohexanediol or cyclodecanediol, and polyalkylene ether glycols such as polyethylene ether glycol, polyoxytrimethylene ether glycol, and polypropylene ether glycol.

The biphenol (3) used as a raw material and another multivalent hydroxy compound used as necessary are dissolved in the epihalohydrin in an amount corresponding to typically 1.0 to 10.0 equivalents, preferably 2.5 to 8.5 equivalents, or more preferably 4.0 to 7.0 equivalents per equivalent of the hydroxyl group of the total multivalent hydroxy compound which is the total of the biphenol (3) and the multivalent hydroxy compound, to make a homogeneous solution. A case in which the amount of the epihalohydrin is not less than the lower limit of the range described above is preferred because a reaction for increasing a molecular weight can be easily controlled, and the epoxy equivalent of an obtained epoxy resin can be allowed to be suitable. On the other hand, a case in which the amount of the epihalohydrin is not more than the upper limit of the range described above is preferred because production efficiency tends to be improved. Epichlorohydrin, epibromohydrin, or the like is commonly used as the epihalohydrin in this reaction.

Then, while stirring the solution, an alkali metal hydroxide in an amount corresponding to typically 0.5 to 2.0 equivalents, preferably 0.7 to 1.8 equivalents, or more preferably 0.9 to 1.6 equivalents per equivalent of the hydroxyl group of the total multivalent hydroxy compound as a raw material is added in the form of a solid or an aqueous solution, to cause the reaction thereof. A case in which the amount of the alkali metal hydroxide is not less than the lower limit of the range described above is preferred because an unreacted hydroxyl group and a generated epoxy resin are inhibited from reacting with each other to facilitate the control of a reaction for increasing a molecular weight. A case in which the amount

of the alkali metal hydroxide is not more than the upper limit value of the range described above is preferred because impurities are inhibited from being generated by side reaction. Typical examples of the alkali metal hydroxide used here include sodium hydroxide and potassium hydroxide.

This reaction can be carried out under ordinary pressure or reduced pressure, and the temperature of the reaction is preferably 20 to 150°C, more preferably 40 to 100°C, and still more preferably 40 to 80°C. A case in which the reaction temperature is not less than the lower limit of the range described above is preferred because the reaction is allowed to easily proceed, and the reaction is easily controlled. A case in which the reaction temperature is not more than the upper limit of the range described above is preferred because side reaction is inhibited from proceeding, and in particular, chlorine impurities are easily reduced.

The reaction is preferably performed while performing dehydration by a method in which a reaction liquid is removed by azeotropic distillation while keeping predetermined temperature as needed, oil/water separation of a condensate liquid obtained by cooling volatilized vapor is performed, and an oil component obtained by removing water is put back in a reaction system. To suppress rapid reaction, small amounts of alkali metal hydroxide are intermittently or continuously added for preferably 0.1 to 8 hours, more preferably 0.1 to 7 hours, and still more preferably 0.5 to 6 hours. A case in which the time for which the alkali metal hydroxide is added is not less than the lower limit of the range described above is preferred because the reaction can be prevented from rapidly proceeding, and the control of the reaction temperature is facilitated. A case in which the addition time is not more than the upper limit of the range described above is preferred because chlorine impurities are inhibited from being generated. This case is also preferred from the viewpoint of economic efficiency. After the end of the reaction, an insoluble by-product salt is filtered and removed, or removed by washing with water, and an unreacted epihalohydrin is then distilled and removed under reduced pressure, whereby a crude epoxy resin of interest can be obtained.

In this reaction, it is acceptable to use a catalyst such as: a quaternary ammonium salt such as tetramethylammonium chloride or tetraethylammonium bromide; a tertiary amine such as benzyldimethylamine or 2,4,6-tris(dimethylaminomethyl)phenol; an imidazole such as 2-ethyl-4-methylimidazole or 2-phenylimidazole; a phosphonium salt such as ethyltriphenylphosphoniumiodide; or a phosphine such as triphenylphosphine.

Furthermore, in this reaction, it is also acceptable to use an inert organic solvent such as: an alcohol such as ethanol or isopropanol; a ketone such as acetone, methyl ethyl ketone, or methyl isobutyl ketone; an ether such as dioxane or ethylene glycol dimethyl ether; a glycol ether such as methoxypropanol; or an aprotic polar solvent such as dimethylsulfoxide or dimethylformamide.

[Production of Polycrystalline Body of Biphenol Type Diglycidyl Ether]

The polycrystalline body of the biphenol type diglycidyl ether, having the crystal properties described above, can be obtained by treating the crude epoxy resin, produced in the manner described above, by any method of the production methods (1) to (3) described above. Moreover, the crystal properties can be adjusted in preferred ranges by adjusting crystallization conditions, recrystallization treatment, or the conditions of reaction with the alkali metal hydroxide. The crystallization conditions, the recrystallization treatment, or the conditions of reaction with the alkali metal hydroxide may be adjusted singly or in combination.

In other words, for example, an epoxy equivalent can be increased by increasing a temperature, the content of resin in a solvent, or an alkali content. In contrast, an epoxy equivalent can be decreased by decreasing a temperature, the content of resin in a solvent, or an alkali content.

Detailed conditions for producing a polycrystalline body of biphenol type diglycidyl ether are described below. However, reaction time may be increased or decreased depending on the conditions. Therefore, a desired polycrystalline body of biphenol type diglycidyl ether can be obtained by performing appropriate sampling, and analyzing the amount of each component and an epoxy equivalent.

An organic solvent for dissolving an epoxy resin may be used for the reaction between the crude epoxy resin and the alkali metal hydroxide. The organic solvent used in the reaction is not particularly limited, and is preferably the mixed solvent of an aprotic polar solvent and an inert organic solvent other than the aprotic polar solvent in view of production efficiency, handleability, workability, and the like.

Examples of the aprotic polar solvent include dimethylsulfoxide, diethylsulfoxide, dimethylsulfone, sulfolane, dimethylformamide, dimethylacetamide, and hexamethylphosphoramide. These may be used singly, or in admixture of two or more kinds thereof. Among these aprotic polar solvents, dimethylsulfoxide is preferred because of being easily obtained and having an excellent effect.

Examples of the organic solvent used together with the aprotic polar solvent include ketone-based solvents such as methyl ethyl ketone, methyl isobutyl ketone, and cyclohexanone. In view of effects, the ease of post-treatment, and the like, a ketone-based solvent is preferred, and methyl isobutyl ketone is particularly preferred. These may be used singly, or in admixture of two or more kinds thereof.

The aprotic polar solvent and other organic solvent described above are preferably used so that the rate of the

aprotic polar solvent is 1 to 30% by mass, particularly 10 to 20% by mass, with respect to the total thereof.

The amount of the organic solvent used is preferably an amount in which the concentration of the crude epoxy resin is typically 3 to 70% by mass, more preferably an amount in which the concentration is 5 to 50% by mass, and still more preferably an amount in which the concentration is 10 to 45% by mass.

Examples of the alkali metal hydroxide include potassium hydroxide and sodium hydroxide. As the alkali metal hydroxide, an alkali metal hydroxide dissolved in an organic solvent is preferably used.

The amount of the alkali metal hydroxide used is preferably 0.01 parts by mass or more and 0.49 parts by mass or less with respect to 100 parts by mass of the crude epoxy resin in terms of the solid content of the alkali metal hydroxide. The ratio of each component of an obtained epoxy resin, and, in addition, the epoxy equivalent can be easily adjusted in the preferred ranges described above by allowing the amount of the alkali metal hydroxide used to be in such a range. A case in which the amount of the alkali metal hydroxide is not in the range described above may prevent the epoxy resin (1), the epoxy resin (2), and the epoxy resin, including the other components, which are optional components, from being obtained in the desired ranges.

The temperature of the reaction between the crude epoxy resin and the alkali metal hydroxide is preferably 40 to 90°C, and more preferably 50 to 80°C. The time of the reaction is preferably 0.1 to 15 hours, and more preferably 0.3 to 12 hours. A case in which the reaction temperature is not in the range described above may prevent the epoxy resin (1), the desired epoxy resin (2), and the epoxy resin, including the other components, which are optional components, from being obtained.

After the reaction, an excessive alkali metal hydroxide and secondarily characteristic salt are removed by a method such as washing with water, the organic solvent is further removed by distillation under reduced pressure and/or steam distillation, and crystallization is performed under desired crystallization treatment conditions, whereby the polycrystalline body of the biphenol type diglycidyl ether can be obtained.

With regard to the crystallization treatment conditions under which the polycrystalline body of the biphenol type diglycidyl ether is obtained, a pail can made of SUS is preferably used as a container used in the crystallization. The amount of a raw material filled into the pail can is preferably 5 to 20 kg, more preferably 8 to 18 kg, and most preferably 10 to 16 kg. In a case in which the filling amount is not less than the lower limit value of the range described above, change in quality during the crystallization is inhibited from proceeding because a gas area in the pail can is small. In a case in which the filling amount is not more than the upper limit value of the range described above, the filled material is easily extracted from the pail can.

A retention temperature in the crystallization is preferably 8 to 50°C. The retention temperature is more preferably 10 to 40°C, and most preferably 15 to 30°C. In a case in which the retention temperature is not less than lower limit value of the range described above, time for which a content is crystallized can be shortened. In a case in which the retention temperature is not more than the upper limit value of the range described above, a crystalline body having a crystallite size range defined in the present embodiment is obtained.

Retention days in the crystallization are preferably 0.5 to 40 days, more preferably 1 to 30 days, and most preferably 1.5 to 20 days. In a case in which the retention days are not less than the lower limit value of the range described above, a crystalline body having a crystallite size range defined in the present embodiment is obtained. In a case in which the retention days are not more than the upper limit value of the range described above, time for which a content is crystallized can be shortened.

In the organic compound including an epoxy resin, the crystallite size can be adjusted by changing a crystallization rate. Specifically, a crystalline body having a small crystallite size is obtained by increasing the crystallization rate, while a crystalline body having a large crystallite size is obtained by decreasing the crystallization rate. A crystallization method for obtaining the polycrystalline body according to the present embodiment described above is characterized by obtaining a polycrystalline body of biphenol type diglycidyl ether having a sufficiently large crystallite size defined in the present embodiment by carrying out the method under conditions for decreasing the crystallization rate.

Examples of a method of obtaining the crystalline body of the organic compound including an epoxy resin include a method in which shearing stress is added from the outside while heating a molten resin prior to crystallization to accelerate crystallization in order to increase a crystallization rate, and a method utilizing recrystallization in which an organic compound is dissolved in a poor solvent once, and the solution is rapidly cooled to accelerate crystallization. Each of the methods increases a crystallization rate. In contrast, the crystallization method for obtaining the polycrystalline body according to the present embodiment is characterized by decreasing a crystallization rate to increase a crystallite size, as described above.

Accordingly, in the above-described two methods of increasing a crystallization rate, a crystallite size calculated from a peak having a specific diffraction angle (2θ) in a powder X-ray diffraction pattern measured with a CuKα ray is smaller than the crystallite size defined in the present embodiment, and therefore, a crystalline body having the crystallite size range defined in the present embodiment is not obtained.

[Resin Composition Including Polycrystalline Body of Biphenol Type Diglycidyl Ether]

A resin composition according to another embodiment of the present invention is a resin composition including the polycrystalline body of the biphenol type diglycidyl ether described above, and is preferably a resin composition further blended as appropriate with a curing agent, another epoxy resin other than the epoxy resins described above (hereinafter, may be simply referred to as "other epoxy resin"), a curing accelerator, an inorganic filler, a mold release agent, a coupling agent, or the like as needed. In particular, the resin composition preferably includes a curing agent.

[Curing Agent]

Herein, the curing agent refers to a substance contributing to crosslinking reaction and/or chain-lengthening reaction between epoxy groups in an epoxy resin. Herein, a substance that is commonly called "curing accelerator" is regarded as the curing agent as long as the substance contributes to crosslinking reaction and/or chain-lengthening reaction between epoxy groups in an epoxy resin.

The content of the curing agent in the resin composition including the polycrystalline body of the biphenol type diglycidyl ether is not particularly limited, and is preferably 0.01 to 1000 parts by mass, more preferably 0.1 to 1000 parts by mass, still more preferably 500 parts by mass or less, and still more preferably 300 parts by mass or less with respect to 100 parts by mass of the total epoxy resin component as a solid content.

Herein, "solid content" means a component excluding a solvent, and encompasses not only an epoxy resin which is a solid as a property but also a semi-solid or viscous liquid substance. The term "total epoxy resin component" is equivalent to the amount of epoxy resins included in the resin composition including the polycrystalline body of the biphenol type diglycidyl ether described above, and corresponds to the amount of the epoxy resins (an epoxy resin (1), and an epoxy resin (2) and an epoxy resin in another component, which are optional components) in the polycrystalline body of the biphenol type diglycidyl ether in a case in which the resin composition including the polycrystalline body of the biphenol type diglycidyl ether includes only the polycrystalline body of the biphenol type diglycidyl ether. In a case in which the resin composition including the polycrystalline body of the biphenol type diglycidyl ether includes the polycrystalline body of the biphenol type diglycidyl ether and the other epoxy resin, the total epoxy resin component is equivalent to the total amount of the epoxy resin in the polycrystalline body of the biphenol type diglycidyl ether, and the other epoxy resin.

The curing agent is not particularly limited, and each of the curing agents commonly known as epoxy resin curing agents can be used as the curing agent. Examples thereof include: phenolic curing agents; amine-based curing agents such as aliphatic amines, polyether amines, alicyclic amines, and aromatic amines; acid anhydride-based curing agents; amide-based curing agents; tertiary amines; and imidazoles. At least one selected from the group consisting of phenolic curing agents, amine-based curing agents, acid anhydride-based curing agents, and amide-based curing agents is preferred.

Of these, the resin composition including the polycrystalline body of the biphenol type diglycidyl ether includes a phenolic curing agent, whereby the resin composition can have excellent heat resistance, stress resistance, water absorption resistance, flame resistance, and the like. Therefore, the resin composition preferably includes a phenolic curing agent as the curing agent. The resin composition preferably includes an acid anhydride-based curing agent or an amide-based curing agent from the viewpoint of heat resistance and the like. The resin composition preferably includes an amine-based curing agent from the viewpoint of enhancing the glass transformation temperature of the resin composition including the polycrystalline body of the biphenol type diglycidyl ether. It is also preferable to use an imidazole from the viewpoint of allowing curing reaction to sufficiently proceed and improving heat resistance.

The curing agent may be used singly, or in combination of two or more kinds thereof. In the case of using two or more curing agents together, the curing agents may be mixed in advance to prepare and then use a mixed curing agent, or each component of the curing agents may be separately added and simultaneously mixed when each component of the resin composition including the polycrystalline body of the biphenol type diglycidyl ether is mixed.

<Phenolic Curing Agents>

Specific examples of the phenolic curing agents include: various polyphenols such as bisphenol A, bisphenol F, bisphenol S, bisphenol AD, hydroquinone, resorcin, methylresorcin, biphenol, tetramethylbiphenol, dihydroxynaphthalene, dihydroxydiphenyl ether, thiodiphenol, phenol novolac resin, cresol novolac resin, phenol aralkyl resin, biphenyl aralkyl resin, naphthol aralkyl resin, terpene phenol resin, dicyclopentadiene phenol resin, bisphenol A novolac resin, trisphenol methane type resin, naphthol novolac resin, brominated bisphenol A, and brominated phenol novolac resin; polyphenol resins obtained by condensation reaction of various phenols with various aldehydes such as benzaldehyde, hydroxybenzaldehyde, crotonaldehyde, and glyoxal; polyphenol resins obtained by condensation reaction of xylene resin with phenols; cocondensated resins of heavy oils or pitches, phenols, and formaldehydes; and various phenol resins such as phenol/benzaldehyde/xylylene dimethoxide polycondensates, phenol/benzaldehyde/xylylene dihalide polycondensates, phenol/benzaldehyde/4,4'-dimethoxide biphenyl polycondensates, and phenol/benzaldehyde/4,4'-dihalide biphenyl polycondensates.

The phenolic curing agents may be used singly, or in optional combination of two or more kinds thereof at an optional blending ratio.

Among the phenolic curing agents described above, phenol novolac resin (for example, a compound represented by the following Formula (7)), phenol aralkyl resin (for example, a compound represented by the following Formula (8)), biphenyl aralkyl resin (for example, a compound represented by the following Formula (9)), naphthol novolac resin (for example, a compound represented by the following Formula (10)), naphthol aralkyl resin (for example, a compound represented by the following Formula (11)), trisphenol methane type resin (for example, a compound represented by the following Formula (12)), a phenol/benzaldehyde/xylylene dimethoxide polycondensate (for example, a compound represented by the following Formula (13)), a phenol/benzaldehyde/xylylene dihalide polycondensate (for example, a compound represented by the following Formula (13)), a phenol/benzaldehyde/4,4'-dimethoxide biphenyl polycondensate (for example, a compound represented by the following Formula (14)), a phenol/benzaldehyde/4,4'-dihalide biphenyl polycondensate (for example, a compound represented by the following Formula (14)), or the like is preferred from the viewpoint of the heat resistance, curability, and the like of a cured composition.

Of these, phenol novolac resin (for example, a compound represented by the following Formula (7)), phenol aralkyl resin (for example, a compound represented by the following Formula (8)), biphenyl aralkyl resin (for example, a compound represented by the following Formula (9)), a phenol/benzaldehyde/xylylene dimethoxide polycondensate (for example, a compound represented by the following Formula (13)), a phenol/benzaldehyde/xylylene dihalide polycondensate (for example, a compound represented by the following Formula (13)), a phenol/benzaldehyde/4,4'-dimethoxide biphenyl polycondensate (for example, a compound represented by the following Formula (14)), or a phenol/benzaldehyde/4,4'-dihalide biphenyl polycondensate (for example, a compound represented by the following Formula (14)) is particularly preferred.

[Chem 6]

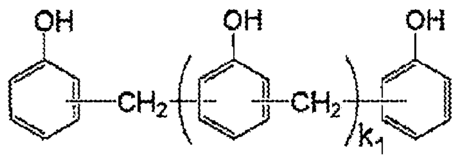

...(7)

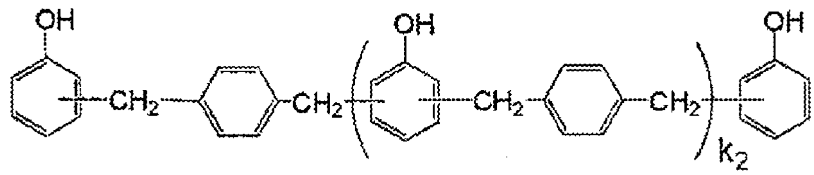

...(8)

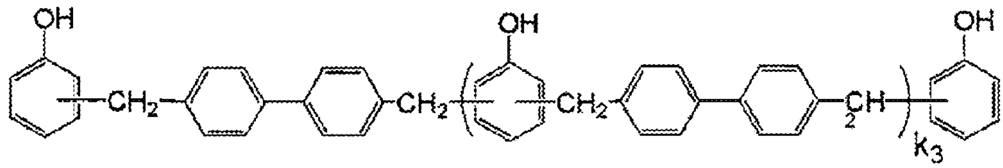

...(9)

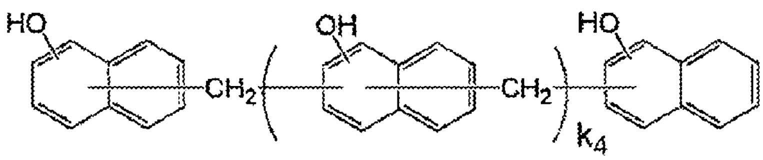

...(10)

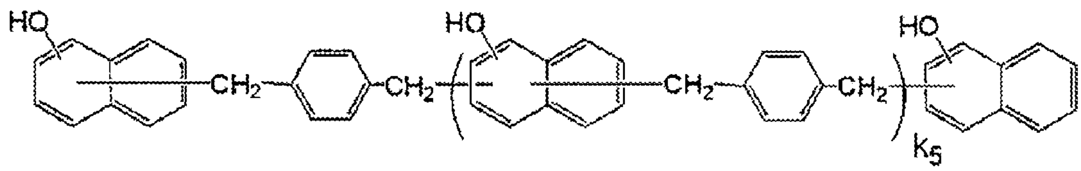

...(11)

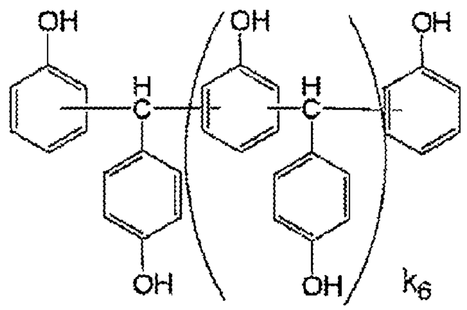

...(12)

In Formulae (7) to (12) described above, each of $k_1$ to $k_6$ independently represents a number of 0 or more, and may be 10 or less, or may be 5 or less.

[Chem 7]

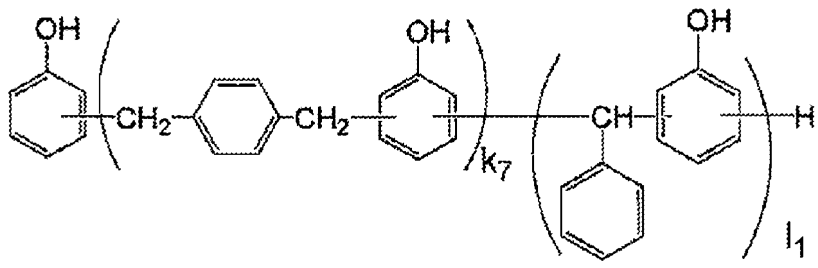

···(13)

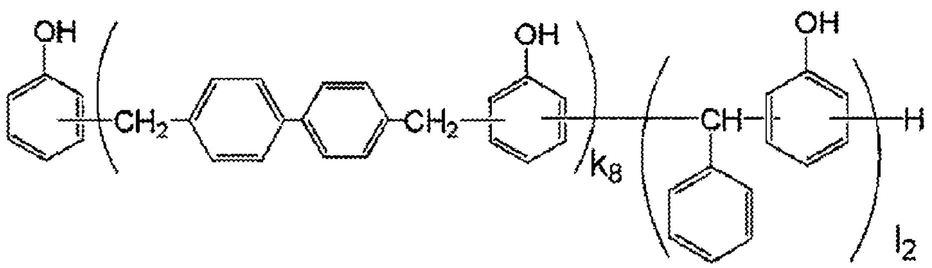

···(14)

In Formulae (13) and (14), each of $k_7$, $k_8$, $l_1$, and $l_2$ independently represents a number of 1 or more, and may be 10 or less, or may be 5 or less.

The amount of the blended phenolic curing agent is preferably 0.1 to 1000 parts by mass, more preferably 500 parts by mass or less, still more preferably 300 parts by mass or less, and particularly preferably 100 parts by mass or less with respect to 100 parts by mass of the total epoxy resin component in the resin composition including the polycrystalline body of the biphenol type diglycidyl ether. A case in which the blending amount is within this range is preferred because an unreacted epoxy group, and a functional group in the curing agent are inhibited from remaining.

<Amine-Based Curing Agents>

Examples of the amine-based curing agents (excluding tertiary amines) include aliphatic amines, polyether amines, alicyclic amines, and aromatic amines.

Examples of the aliphatic amines include ethylenediamine, 1,3-diaminopropane, 1,4-diaminopropane, hexamethylenediamine, 2,5-dimethylhexamethylenediamine, trimethylhexamethylenediamine, diethylenetriamine, iminobispropylamine, bis(hexamethylene)triamine, triethylenetetramine, tetraethylenepentamine, pentaethylenehexamine, N-hydroxyethylethylenediamine, or tetra(hydroxyethyl)ethylenediamine.

Examples of the polyether amines include triethyleneglycoldiamine, tetraethyleneglycoldiamine, diethyleneglycol bis(propylamine), polyoxypropylenediamine, and polyoxypropylene triamine.

Examples of the alicyclic amines include isophoronediamine, menthenediamine, N-aminoethylpiperazine, bis(4-amino-3-methyldicyclohexyl)methane, bis(aminomethyl)cyclohexane, 3,9-bis(3-aminopropyl)-2,4,8,10-tetraoxaspiro(5,5)undecane, and norbornenediamine.

Examples of the aromatic amines include tetrachloro-p-xylenediamine, m-xylenediamine, p-xylenediamine, m-phenylenediamine, o-phenylenediamine, p-phenylenediamine, 2,4-diaminoanisole, 2,4-toluenediamine, 2,4-diaminodiphenylmethane, 4,4'-diaminodiphenylmethane, 4,4'-diamino-1,2-diphenylethane, 2,4-diaminodiphenylsulphone, 4,4'-diaminodiphenylsulphone, m-aminophenol, m-aminobenzylamine, benzyldimethylamine, 2-(dimethylaminomethyl)phenol, triethanolamine, methylbenzylamine, $\alpha$-(m-aminophenyl)ethylamine, $\alpha$-(p-aminophenyl)ethylamine, diaminodiethyl dimethyl diphenylmethane, and $\alpha,\alpha'$-bis(4-aminophenyl)-p-diisopropylbenzene.

The amine-based curing agents may be used singly, or in optional combination of two or more kinds thereof at an optional blending ratio.

The amount of the blended amine-based curing agent is preferably set so that the equivalent ratio of a functional group in the curing agent to an epoxy group in the total epoxy resin component included in the resin composition including the polycrystalline body of the biphenol type diglycidyl ether is in a range of 0.8 to 1.5. A case in which the blending amount is in this range is preferred because an unreacted epoxy group, and a functional group in the curing agent are inhibited from remaining.

<Tertiary Amines>

Examples of the tertiary amines include 1,8-diazabicyclo(5,4,0)undecene-7, triethylenediamine, benzyldimethylamine, triethanolamine, dimethylaminoethanol, and tris(dimethylaminomethyl)phenol.
The tertiary amines may be used singly, or in optional combination of two or more kinds thereof at an optional blending ratio.
The amount of the blended tertiary amine is preferably set so that the equivalent ratio of a functional group in the curing agent to an epoxy group in the total epoxy resin component included in the resin composition including the polycrystalline body of the biphenol type diglycidyl ether is in a range of 0.8 to 1.5. A case in which the blending amount is in this range is preferred because an unreacted epoxy group, and a functional group in the curing agent are inhibited from remaining.

<Acid Anhydride-Based Curing Agents>

Examples of the acid anhydride-based curing agents include acid anhydrides, and modified products of acid anhydrides.
Examples of the acid anhydrides include phthalic anhydrides, trimellitic anhydrides, pyromellitic anhydrides, benzophenonetetracarboxylic anhydrides, dodecenylsuccinic anhydrides, polyadipic anhydrides, polyazelaic polyanhydrides, polysebacic anhydrides, poly(ethyloctadecanedioic)anhydrides, poly (phenylhexadecanedioic) anhydrides, tetrahydrophthalic anhydrides, methyltetrahydrophthalic anhydrides, methylhexahydrophthalic anhydrides, hexahydrophthalic anhydrides, methylhimic anhydrides, trialkyltetrahydrophthalic anhydrides, methylcyclohexenedicarboxylic anhydrides, methylcyclohexenetetracarboxylic anhydrides, ethyleneglycolbistrimellitate dianhydrides, HET anhydrides, nadic anhydrides, methylnadic anhydrides, 5-(2,5-dioxotetrahydro-3-furanyl)-3-methyl-3-cyclohexane-1,2-dicarboxylic anhydrides, 3,4-dicarboxy-1,2,3,4-tetrahydro-1-naphthalenesuccinic dianhydrides, and 1-methyl-dicarboxy-1,2,3,4-tetrahydro-1-naphthalenesuccinic dianhydrides.
Examples of the modified products of acid anhydrides include products obtained by modifying the above-described acid anhydrides with glycols. Examples of the glycols that can be used in the modification include: alkylene glycols such as ethylene glycol, propylene glycol, and neopentyl glycol; and polyether glycols such as polyethylene glycol, polypropylene glycol, and polytetramethylene ether glycol. Furthermore, copolymerized polyether glycols of two or more glycols and/or polyether glycols of the glycols can be used.
In the modified product of an acid anhydride, the modification with 0.4 mol or less of a glycol with respect to 1 mol of the acid anhydride is preferred. In a case in which a modification amount is not more than the upper limit value of the range described above, an excessive increase in the viscosity of an epoxy resin composition tends to be prevented to achieve favorable workability, and the rate of curing reaction with an epoxy resin also tends to be favorable.
The acid anhydride-based curing agents may be used singly, or in optional combination of two or more kinds thereof in optional blending amounts.
In the case of using such an acid anhydride-based curing agent, the amount of the blended acid anhydride-based curing agent is preferably set so that the equivalent ratio of a functional group in the curing agent to an epoxy group in the total epoxy resin component in the resin composition including the polycrystalline body of the biphenol type diglycidyl ether is in a range of 0.8 to 1.5. A case in which the blending amount is in this range is preferred because an unreacted epoxy group, and a functional group in the curing agent are inhibited from remaining.

<Amide-Based Curing Agents>

Examples of the amide-based curing agents include dicyandiamide or derivatives thereof, and polyamide resins. The amide-based curing agents may be used singly, or may be mixed and used in optional combination of two or more kinds thereof at an optional ratio.
In the case of using such an amide-based curing agent, with regard to the amount of the blended amide-based curing agent, the amide-based curing agent is preferably 0.1 to 20% by mass with respect to the total of the total epoxy resin component and the amide-based curing agent in the resin composition including the polycrystalline body of the biphenol type diglycidyl ether. A case in which the blending amount is in the range is preferred because an unreacted epoxy group, and a functional group in the curing agent is inhibited from remaining.

<Imidazoles>

Examples of the imidazoles include 2-phenylimidazole, 2-ethyl-4(5)-methylimidazole, 2-phenyl-4-methylimidazole, 1-benzyl-2-methylimidazole, 1-benzyl-2-phenylimidazole, 1-cyanoethyl-2-undecylimidazole, 1-cyano-2-phenylimidazole, 1-cyanoethyl-2-undecylimidazoletrimellitate, 1-cyanoethyl-2-phenylimidazoliumtrimellitate, 2,4-diamino-6-[2'-methylimidazolyl-(1')]-ethyl-s-triazine, 2,4-diamino-6-[2'-ethyl-4'-methylimidazolyl-(1')]-ethyl-s-triazine, adducts of 2,4-diamino-6-[2'-methylimidazolyl-(1')]-ethyl-s-triazine isocyanuric acid, adducts of 2-phenylimidazole isocyanuric acid, 2-phenyl-4,5-dihydroxymethylimidazole, 2-phenyl-4-methyl-5-hydroxymethylimidazole, and adducts of epoxy resins and the imidazoles described above.

The imidazoles have catalytic abilities, and therefore, can also be commonly classified as curing accelerators. Herein, however, the imidazoles are classified as curing agents.

The imidazoles may be mixed and used singly, or in optional combination of two or more kinds thereof at an optional ratio.

In the case of using such an imidazole, the amount of the blended imidazole is preferably 0.1 to 20% by mass with respect to the total of the total epoxy resin component and the imidazole in the resin composition including the polycrystalline body of the biphenol type diglycidyl ether. A case in which the blending amount is in the range is preferred because an unreacted epoxy group, and a functional group in the curing agent are inhibited from remaining.

<Other Curing Agents>

In the resin composition including the polycrystalline body of the biphenol type diglycidyl ether, other curing agents except the curing agents described above can be used. The other curing agents are not particularly limited, and all the curing agents that are commonly known as curing agents for epoxy resins can be used as the other curing agents.

The other curing agents may be used singly, or in combination of two or more kinds thereof.

[Other Epoxy Resins]

The resin composition including the polycrystalline body of the biphenol type diglycidyl ether can include still another epoxy resin except the polycrystalline body of the biphenol type diglycidyl ether. The inclusion of the other epoxy resin enables improvement in the heat resistance, stress resistance, water absorption resistance, flame resistance, or the like of the resin composition.

Such other epoxy resins that can be used in the resin composition including the polycrystalline body of the biphenol type diglycidyl ether correspond to all the epoxy resins other than the above-described epoxy resins (the epoxy resin (1), and the epoxy resin (2) as an optional component).

Specific examples of the other epoxy resins include bisphenol A type epoxy resins, trisphenol methane type epoxy resins, anthracene type epoxy resins, phenol-modified xylene resin type epoxy resins, bisphenol cyclododecyl type epoxy resins, bisphenol diisopropylidene resorcin type epoxy resins, bisphenol F type epoxy resins, bisphenol AD type epoxy resins, hydroquinone type epoxy resins, methylhydroquinone type epoxy resins, dibutylhydroquinone type epoxy resins, resorcin type epoxy resins, methylresorcin type epoxy resins, biphenol type epoxy resins, tetramethylbiphenol type epoxy resins other than the epoxy resins (1) and (2) described above, tetramethyl bisphenol F type epoxy resins, dihydroxydiphenyl ether type epoxy resins, epoxy resins derived from thiodiphenols, dihydroxynaphthalene type epoxy resins, dihydroxyanthracene type epoxy resins, dihydroxydihydroanthracene type epoxy resins, dicyclopentadiene type epoxy resins, epoxy resin derived from dihydroxystilbene, phenol novolac type epoxy resins, cresol novolac type epoxy resins, bisphenol A novolac type epoxy resins, naphthol novolac type epoxy resins, phenol aralkyl type epoxy resins, naphthol aralkyl type epoxy resins, biphenyl aralkyl type epoxy resins, terpene phenol type epoxy resins, dicyclopentadiene phenol type epoxy resins, epoxy resins derived from condensates of phenolhydroxybenzaldehyde, epoxy resins derived from condensates of phenol-crotonaldehyde, epoxy resins derived from condensates of phenol-glyoxal, epoxy resins derived from cocondensated resins of heavy oils or pitches, phenols, and formaldehydes, epoxy resins derived from diaminodiphenyl methane, epoxy resins derived from aminophenol, epoxy resins derived from xylenediamine, epoxy resins derived from methyl hexahydrophthalic acid, and epoxy resins derived from dimer acids.

These may be used singly, or in optional combination of two or more kinds thereof at an optional blending ratio.

Among the other epoxy resins described above, bisphenol A type epoxy resins, tetramethylbiphenol type epoxy resins other than the epoxy resins (1) and (2), 4,4'-biphenol type epoxy resins, biphenyl aralkyl type epoxy resins, phenol aralkyl type epoxy resins, dihydroxyanthracene type epoxy resins, dicyclopentadiene type epoxy resins, orthocresol novolac type epoxy resins, and trisphenol methane type epoxy resins are particularly preferred from the viewpoint of the flowability of the composition, in addition, the heat resistance, water absorption resistance, and flame resistance of a cured product, and the like.

In a case in which the resin composition including the polycrystalline body of the biphenol type diglycidyl ether includes the other epoxy resin described above, the content thereof is preferably 0.01 to 60 parts by mass, more preferably 40 parts by mass or less, still more preferably 30 parts by mass or less, and particularly preferably 20 parts by mass or less, and more preferably 1 part by mass or more with respect to 100 parts by mass of the total epoxy resin component in the composition.

[Curing Accelerators]

The resin composition including the polycrystalline body of the biphenol type diglycidyl ether preferably includes a curing accelerator. The inclusion of the curing accelerator enables a shortening of curing time, and a decrease in curing temperature, whereby a desired cured product can be easily obtained.
The curing accelerator is not particularly limited, and specific examples thereof include phosphorus-based compounds such as organic phosphines and phosphonium salts, tetraphenyl boron salts, organic acid dihydrazides, and boron halide amine complexes.
Examples of the phosphorus-based compounds that can be used as such curing accelerators include: organic phosphines such as triphenylphosphine, diphenyl(p-tolyl)phosphine, tris(alkylphenyl)phosphine, tris(alkyloxyphenyl)phosphine, tris(alkyl-alkyloxyphenyl)phosphine, tris(dialkylphenyl)phosphine, tris(trialkylphenyl)phosphine, tris(tetraalkylphenyl)phosphine, tris(dialkoxyphenyl)phosphine, tris(trialkoxyphenyl)phosphine, tris(tetraalkyloxyphenyl)phosphine, trialkylphosphine, dialkylarylphosphine, and alkyldiarylphosphine; complexes of these organic phosphines and organic borons; and compounds obtained by adding the organic phosphines, a maleic anhydride, a quinone compound such as 1,4-benzoquinone, 2,5-toluquinone, 1,4-naphthoquinone, 2,3-dimethylbenzoquinone, 2,6-dimethylbenzoquinone, 2,3-dimethoxy-5-methyl-1,4-benzoquinone, 2,3-dimethoxy-1,4-benzoquinone, or phenyl-1,4-benzoquinone, or a compound such as diazophenylmethane.
Among the curing accelerators mentioned above, organic phosphines and phosphonium salts are preferred, and organic phosphines are most preferred.
The curing accelerators mentioned above may be used singly, or may be mixed and used in optional combination of two or more kinds thereof at an optional ratio.
The content of the curing accelerator is preferably in a range of 0.1 by mass or more and 20 parts by mass or less, more preferably 0.5 parts by mass or more, and still more preferably 1 part by mass or more, and more preferably 15 parts by mass or less, and still more preferably 10 parts by mass or less, with respect to 100 parts by mass of the total epoxy resin component in the resin composition including the polycrystalline body of the biphenol type diglycidyl ether. A case in which the content of the curing accelerator is not less than the lower limit value of the range described above is preferred because a favorable curing acceleration effect can be obtained. A case in which the content of the curing accelerator is not more than the upper limit value of the range described above is preferred because a desired cured product property is easily obtained.

[Inorganic Fillers]

The resin composition including the polycrystalline body of the biphenol type diglycidyl ether can be blended with an inorganic filler. Examples of the inorganic filler include fused silica, crystalline silica, glass powder, alumina, calcium carbonate, calcium sulfate, talc, and boron nitride. These may be used singly, or in optional combination of two or more kinds thereof at an optional blending ratio. Of these, crushed and/or spherical, fused and/or crystalline silica powder fillers are preferred in the case of use thereof in applications for sealing semiconductors.
Use of such an inorganic filler enables the thermal expansion coefficient of a semiconductor sealing material to be allowed to approach that of an internal silicon chip or lead frame in the case of using the resin composition including the polycrystalline body of the biphenol type diglycidyl ether as the semiconductor sealing material. In addition, the water absorption of the whole semiconductor sealing material can be reduced, and therefore, solder cracking resistance can be improved.
The average particle diameter of the inorganic filler is typically 1 to 50 $\mu$m, preferably 1.5 to 40 $\mu$m, and more preferably 2 to 30 $\mu$m. A case in which the average particle diameter is not less than the lower limit value of the range described above is preferred because melt viscosity is prevented from being excessively increased, and flowability is inhibited from deteriorating.
A case in which the average particle diameter of the inorganic filler is not more than the upper limit value of the range described above is preferred because a narrow gap of a die is inhibited from being clogged with the filler in molding, and the filling properties of the material are easily improved.
In the case of using the inorganic filler in the resin composition including the polycrystalline body of the biphenol type diglycidyl ether, the amount of the inorganic filler blended in the epoxy resin composition is preferably 60 to 95% by mass.

[Mold Release Agents]

The resin composition including the polycrystalline body of the biphenol type diglycidyl ether can be blended with a mold release agent. As the mold release agent, for example, a natural wax such as carnauba wax, a synthetic wax such as polyethylene wax, a higher fatty acid such as stearic acid or zinc stearate, or a metallic salt thereof, or a hydrocarbon-based mold release agent such as paraffin can be used. These may be used singly, or in optional combination of two or more kinds thereof at an optional blending ratio.

In the case of blending the resin composition including the polycrystalline body of the biphenol type diglycidyl ether with the mold release agent, the blending amount of the mold release agent is preferably 0.1 to 5.0 parts by mass, more preferably 0.5 to 3.0 parts by mass, with respect to 100 parts by mass of the total epoxy resin component in the resin composition. A case in which the blending amount of the mold release agent is in the range described above is preferred because favorable mold release characteristics can be exhibited while maintaining the curing characteristics of the resin composition.

[Coupling Agents]

The resin composition including the polycrystalline body of the biphenol type diglycidyl ether is preferably blended with a coupling agent. The coupling agent is preferably used together with an inorganic filler. The blending of the coupling agent enables improvement in adhesiveness between the inorganic filler and an epoxy resin which is a matrix. Examples of the coupling agent include silane coupling agents and titanate coupling agents.

Examples of the silane coupling agents include: epoxysilanes such as γ-glycidoxypropyltrimethoxysilane, γ-glycidoxypropyltriethoxysilane, and β-(3,4-epoxycyclohexyl)ethyltrimethoxysilane; aminosilanes such as γ-aminopropyltriethoxysilane, N-β(aminoethyl)γ-aminopropyltrimethoxysilane, N-β(aminoethyl)γ-aminopropylmethyldimethoxysilane, γ-aminopropyltrimethoxysilane, and γ-ureidopropyltriethoxysilane; mercaptosilanes such as 3-mercaptopropyltrimethoxysilane; vinylsilanes such as p-styryltrimethoxysilane, vinyltrichlorosilane, vinyltris(β-methoxyethoxy)silane, vinyltrimethoxysilane, vinyltriethoxysilane, and γ-methacryloxypropyltrimethoxysilane; and epoxy-based, amino-based, or vinyl-based polymer-type silanes.

Examples of the titanate coupling agents include isopropyl triisostearoyltitanate, isopropyl tri(N-aminoethylaminoethyl)titanate, diisopropyl bis(dioctylphosphate)titanate, tetraisopropyl bis(dioctylphosphite)titanate, tetraoctyl bis(ditridecylphosphite)titanate, tetra(2,2-diallyloxymethyl-1-butyl)bis(ditridecyl)phosphitetitanate, bis(dioctylpyrophosphate)oxyacetatetitanate, and bis(dioctylpyrophosphate)ethylenetitanate.

These coupling agents may be used singly, or may be mixed and used in optional combination of two or more kinds thereof at an optional ratio.

In the case of using the coupling agent in the resin composition including the polycrystalline body of the biphenol type diglycidyl ether, the blending amount thereof is preferably 0.1 to 3.0 parts by mass with respect to 100 parts by mass of the total epoxy resin component. A case in which the blending amount of the coupling agent is not less than the lower limit value of the range described above is preferred because the effect of improving adhesiveness between the inorganic filler and the epoxy resin which is a matrix due to the blending of the coupling agent tends to be improved. A case in which the blending amount of the coupling agent is not more than the upper limit value of the range described above is preferred because the coupling agent is inhibited from bleeding from an obtained cured product.

[Other Blending Components]

The resin composition including the polycrystalline body of the biphenol type diglycidyl ether can be blended with a component (herein, may be referred to as "other blending component") other than the above-described components. Examples of such other blending components include flame retardants, plasticizers, reactive diluents, and pigments. These can be optionally blended as appropriate within the range in which the effects of the present invention can be obtained. The resin composition may be blended with a substance other than the above-described components mentioned as the other blending components.

Examples of the flame retardants include: halogen-based flame retardants such as brominated epoxy resins and brominated phenol resins; antimony compounds such as antimony trioxide; phosphorus-based flame retardants such as red phosphorus, phosphate esters, and phosphines; nitrogen-based flame retardants such as melamine derivatives; and inorganic flame retardants such as aluminum hydroxide and magnesium hydroxide.

[Cured Product]

A method of curing the resin composition including the polycrystalline body of the biphenol type diglycidyl ether is not particularly limited.

A cured product which is another embodiment of the present invention is the cured product of a resin composition including a polycrystalline body of biphenol type diglycidyl ether, and can be commonly obtained by curing the above-described resin composition by thermal curing reaction by heating. In the thermal curing reaction, a curing temperature is preferably selected as appropriate depending on the kind of a curing agent used. For example, the curing temperature is typically 130 to 300°C in the case of using a phenolic curing agent. The curing temperature can also be decreased by

adding a curing accelerator to such curing agents. Reaction time is preferably 1 to 20 hours, more preferably 2 to 18 hours, and still more preferably 3 to 15 hours. A case in which the reaction time is not less than the lower limit value of the range described above is preferred because curing reaction tends to sufficiently easily proceed. A case in which the reaction time is not more than the upper limit value of the range described above is preferred because degradation due to heating, and an energy loss in the heating are easily reduced.

[Applications]

The cured product of the resin composition including the polycrystalline body of the biphenol type diglycidyl ether absorbs a little water, is excellent in thermal cracking resistance in molding, and in electrical reliability in the use of the cured product for a long period, and can be preferably used as an electric and electronic material.

The resin composition including the polycrystalline body of the biphenol type diglycidyl ether, and the cured product thereof can be used so as to be preferably included in the electric and electronic material described above. The resin composition and the cured product thereof can be effectively used in any application as long as the physical properties described above are demanded in the applications. For example, the resin composition and the cured product thereof can be preferably included and used in a field such as: an optical field for an optical material or the like; the field of a paint such as a paint for an automobile, such as an electrodeposition paint for an automobile, a heavy duty protective paint for a ship or a bridge, or a paint for internal lining of a beverage can; an electric and electronic field for a composite material, a laminate, a semiconductor sealing material, a liquid insulation sealing material, an insulating powder paint, a material for coil impregnation, or the like; or a civil engineering, architecture, or adhesive agent field for a seismically resistant material for a bridge, a concrete reinforcing material, a floor material for a building, a lining material for a water supply facility, a drainage or permeable pavement material, or an adhesive agent for a structure, a vehicle, or an aircraft. Of these, the resin composition and the cured product thereof can be preferably included and used particularly in the electric and electronic field. Specifically, the resin composition and the cured product thereof are useful as electric and electronic components.

In a case in which the cured product of the resin composition including the polycrystalline body of the biphenol type diglycidyl ether is used in each application, a cured product that has been cured may be used in a material in each application, or a cured product, cured in a production step in each of the applications described above, may be used.

[Laminate]

Examples of a method of producing a laminate by using the resin composition including the polycrystalline body of the biphenol type diglycidyl ether include a method of producing a laminate by heating and pressurizing a layered product including a prepreg obtained by impregnating a fibrous base material with a resin varnish including the resin composition including the polycrystalline body of the biphenol type diglycidyl ether, to cure the layered product.

More specifically, the fibrous base material is impregnated with the resin varnish, and dried to remove a solvent and to make a prepreg. This prepreg and another base material used as necessary are layered to form a layered product. The layered product is heated and pressurized to cure the layered product, and the laminate can be obtained.

The number of layered prepregs in the layered product may be one, or may be two or more. In the layered product, another base material except the prepreg may be layered. Examples of the other base materials include metal foils such as copper foil.

Examples of fibers included in the fibrous base material include: inorganic fibers such as glass fibers, carbon fibers, ceramic fibers, and stainless steel fibers; natural fibers such as cotton, hemp, and paper; and synthetic organic fibers such as polyester resins and polyamide resins. These fibers may be used singly, or in combination of two or more kinds thereof.

The form of the fibrous base material is not particularly limited, and examples thereof include short fibers, yarns, mats, and sheets.

The amount of the resin varnish with which the fibrous base material is impregnated is not particularly limited. For example, the solid amount of the resin varnish with which the fibrous base material is impregnated is set to be around 30 to 50 parts by mass with respect to 100 parts by mass of the fibrous base material.

Heating temperature in the case of heating and pressurizing the layered product is preferably the curing temperature of the resin composition described above. A pressurization condition is preferably 2 to 20 $kN/m^2$.

The laminate produced in such a manner includes a fiber-reinforced resin layer including the fibrous base material and the cured product of the resin varnish. The number of the fiber-reinforced resin layers included in the laminate may be one, or may be two or more. As described above, the laminate may include a metal foil layer such as copper foil.

[Sealing Material]

In the case of using the resin composition including the polycrystalline body of the biphenol type diglycidyl ether

as a sealing material, the form of the sealing material to which the resin composition is applied is not particularly limited, and, for example, a form similar to a form adopted in a known semiconductor or the like can be adopted.

In a method of forming the sealing material by using the resin composition including the polycrystalline body of the biphenol type diglycidyl ether, for example, a transfer molding method, a compression molding method, or the like can be used.

## EXAMPLES

The present invention is more specifically described below with reference to Examples. However, the present invention is not limited at all to the following Examples. The values of various production conditions and evaluation results in the following Examples have meanings as the preferred values of upper or lower limits in aspects of the present invention, and preferred ranges may be ranges defined by the values of the upper or lower limits and the values in the following Examples or combinations of the values in Examples.

[Raw Materials Used, and the Like]

Raw materials used in the following Examples and Comparative Examples, and the structural formulae of reaction products and the like are as follows.

Epoxy Resin (1-1)

[Chem 8]

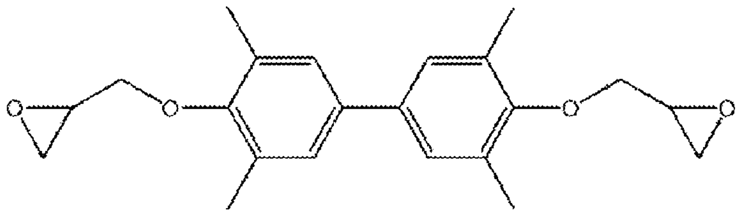

(1-1)

Epoxy Resin (2-1)

[Chem 9]

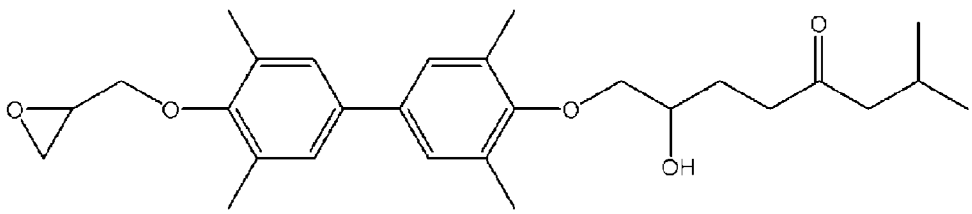

(2-1)

Tetramethylbiphenol (3-1) (Manufactured by Mitsubishi Chemical Corporation)

[Chem 10]

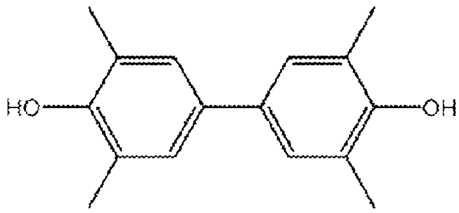

(3-1)

[Measurement and Evaluation Methods]

Methods of measuring and evaluating the physical properties and the like of polycrystalline bodies or epoxy resins obtained in the following Examples and Comparative Examples are as follows.

<Epoxy Equivalent>

The epoxy equivalent of a polycrystalline body is defined as "mass of epoxy resin including 1 equivalent of epoxy group". Such an epoxy equivalent was measured according to JIS K7236.

<Powder X-ray Diffraction (XRD)>

XRD measurement of a powder sample was performed in a concentration optical system using CuKα rays by X'Pert Pro MPD manufactured by PANalytical.

In XRD analysis software JADE Pro, a straight line connecting both ends was removed as a background in each of the following regions, profile fitting was then performed, and a diffraction peak angle (2θ) and a half peak width (β) were derived.

Peak 1: 8.9° to 9.3°, peak 2: 15.1° to 15.5°, peak 3: 19.4° to 19.8°, peak 4: 24.9° to 25.3°

The crystallite size was calculated from the following Equation (A) (Scherrer equation) using a half peak width β obtained by correcting peak broadening resulting from an apparatus.

$$D = K\lambda/\beta \cos \theta \qquad (A)$$

In (Equation A), each symbol is as follows.

D: Crystallite size (Å)
K: Scherrer constant K = 0.9
λ: X-ray wavelength λ = 1.54059 (Å)
β: Half peak width (rad)
θ: Bragg angle (rad)

Here, β was calculated from the following Equation (B) using an actually measured half peak width $\beta_0$ and a half peak width $\beta_i$ resulting from an apparatus. A half peak width calculated from the diffraction pattern of an Si powder (NIST640c) was used as $\beta_i$.

$$\beta^2 = \beta_0{}^2 - \beta_i{}^2 \qquad (B)$$

<Composition of Polycrystalline Body of Biphenol Type Diglycidyl Ether>

The content rate of an epoxy resin (1-1) or (2-1) in a polycrystalline body of biphenol type diglycidyl ether was calculated as a rate (% by mass) obtained by performing LC analysis under the following conditions by the following apparatus on the basis of JIS K0124, and dividing % by area in each area of an LC chart represented by the epoxy resin (1-1), the epoxy resin (2-1), and other components by % by area of the total of the epoxy resin (1-1), the epoxy resin (2-1), and the other components.

Apparatus: high performance liquid chromatography Waters 2690 manufactured by Waters, Column: TSKgel ODS-120A manufactured by TOSOH SILICA CORPORATION (column dimension: 4.6 mm I.D. × 15 cm)

Eluent: acetonitrile/water = 30/70 allowed to be 100/0 for 60 minutes
Gradient analysis flow rate: 1 mL/min
Detector: UV (280 nm)
Temperature: 35°C
Sample concentration: 0.1%
Injection amount: 10 μL
Software for analyzing peak area: Empower 2 manufactured by Waters

<Melting Point>

The melting point of the polycrystalline body was measured using a differential scanning calory measurement instrument (manufactured by Hitachi High-Tech Science Corporation). A temperature at the endothermic peak top in the case of increasing the temperature of 5 mg of sample at 10°C/min was adopted as the melting point. In the case of observing a plurality of peak tops, temperatures at all the peak tops are listed in increasing order of temperature in Table 1.

<Measurement of Melt Viscosity>

The polycrystalline body of the biphenol type diglycidyl ether was melted on the hot plate of a cone-plate viscometer (manufactured by Tokai Yagami Co., Ltd.) adjusted to 150°C, and the melt viscosity (poise) of the polycrystalline body was measured at a rotation speed of 750 rpm.

<Evaluation of Crystallization Days>

The epoxy resin was crystallized in an 18-L pail can, as described in each of Examples and Comparative Examples described later. Days before the polycrystalline body was obtained were regarded as crystallization days, which are set forth in Table 1. A case in which the crystallization days were 45 days or less was evaluated as obtainment of the polycrystalline body in a short period. In each of Examples and Comparative Examples, treatment of the crystallization (treatment of retention at 20°C) in itself was performed for 60 days. The crystallization days set forth in Table 1 were days before the polycrystalline body was obtained.

<Grinding Treatment>

The polycrystalline body of biphenol type diglycidyl ether, obtained by the crystallization in the 18-L pail can in the evaluation of the crystallization days described above, was extracted from the 18-L pail can, and powderized using a grinding machine (FEATHER MILL FM-2, manufactured by HOSOKAWA MICRON CORPORATION), and the grindability thereof was determined according to the following criteria.
A case in which no adhesion was observed in the apparatus in the grinding was evaluated as "A".
A case in which adhesion was observed in the apparatus in the grinding, or a case in which it was impossible to obtain the crystalline body and to perform the grinding was evaluated as "B".

<Measurement of Particle Diameter>

The particle diameters of the ground products obtained by performing the grinding treatment described above were measured, and the average particle diameter (μm), D10 (μm), D50 (μm), and D90 (μm) thereof were calculated. In a case in which the particle diameter was less than 50 μm, the measurement was performed by a laser scattering method (apparatus: 3000EXII manufactured by MicrotracBEL Corp., dispersion medium: water). In a case in which the particle diameter was 50 μm or more, the measurement was performed by a sieve method (apparatus: Micro Vibro Sifter M-3T manufactured by TSUTSUI RIKAGAKU KIKAI CO., LTD., openings of sieves: 32, 63, 125, 250, 500, 1000, 2000, and 4000 μm, vibration condition; 2 minutes at scale 5).
The measurement was performed based on parameters according to the particle diameters, only in Examples 1 to 4.

[Production and Evaluation of Polycrystalline Body of Biphenol Type Diglycidyl Ether]

[Production Example]

In an autoclave equipped with a thermometer, a stirring apparatus, and a cooling pipe, and having an inner capacity of 10 L, 900 g of raw material phenol: tetramethylbiphenol (3-1) (manufactured by Mitsubishi Chemical

Corporation), 4473 g of epichlorohydrin, 1741 g of isopropyl alcohol, and 462 g of water were loaded, and homogeneously dissolved by increasing the temperature thereof to 40°C, followed by dripping 713 g of 48.5% by mass aqueous sodium hydroxide solution for 90 minutes. The temperature was increased from 40°C to 65°C for 90 minutes simultaneously with the dripping. Then, the resultant was retained at 65°C for 30 minutes to complete the reaction, 1340 g of warm water at 65°C was added to dissolve a by-product salt and excessive sodium hydroxide, and the solution was left to stand at 65°C for 1 hour. After the standing, a water layer was extracted from an oil layer and the water layer separated from each other. Then, epichlorohydrin was completely removed under reduced pressure at 150°C to obtain a crude epoxy resin.

Then, 1974 g of methyl isobutyl ketone was loaded, the temperature was increased to 65°C to homogeneously dissolve the resultant, 28 g of 48.5% by mass aqueous sodium hydroxide solution was then loaded, and reacted for 60 minutes, and water washing was then performed four times by using 2000 g of water. Then, methyl isobutyl ketone was completely removed under reduced pressure at 150°C to obtain an epoxy resin.

[Example 1]

An epoxy resin was obtained by an operation similar to that in the production example described above. Then, the crystallization operation of the obtained epoxy resin was performed three times in propylene glycol monomethyl ether. The above-described operation was performed plural times until 10 kg of epoxy resin was obtained. Then, the propylene glycol monomethyl ether was completely removed under reduced pressure at 150°C, and a predetermined amount of the resultant was filled into an 18-L pail can, warmed at 150°C for 6 hours, and then crystallized at 20°C for 60 days to obtain a polycrystalline body of biphenol type diglycidyl ether of Example 1 (production method (2)). With regard to the epoxy resin filled into the pail can, crystallization days before the polycrystalline body was obtained by the above-described method were evaluated. Each measurement and evaluation described above were performed using the polycrystalline body.

These results are set forth in Table **1.**

[Example 2]

By an operation similar to that in the production example described above, the above-described operation was performed plural times until 10 kg of epoxy resin was obtained. A predetermined amount of the obtained epoxy resin was filled into an 18-L pail can, warmed at 150°C for 6 hours, and then crystallized at 20°C for 60 days to obtain a polycrystalline body of biphenol type diglycidyl ether of Example 2 (production method (1)). With regard to the epoxy resin filled into the pail can, crystallization days before the polycrystalline body was obtained by the above-described method were evaluated. Each measurement and evaluation described above were performed using the polycrystalline body.

These results are set forth in Table **1.**

[Example 3]

An epoxy resin was obtained by an operation similar to that in the production example described above. Then, in an autoclave equipped with a thermometer, a stirring apparatus, and a cooling pipe, and having an inner capacity of 10 L, 2500 g of the obtained epoxy resin, 3000 g of methyl isobutyl ketone, and 750 g of dimethylsulfoxide were loaded, and homogeneously dissolved by increasing a reaction temperature to 65°C, followed by adding 123 g of 8% by mass potassium hydroxide/isopropanol solution to perform reaction for 1.5 hours. Then, 750 g of methyl isobutyl ketone was added, and water washing was performed four times by using 1000 g of water. Then, water washing was performed four times by using 1000 g of water. Then, methyl isobutyl ketone was completely removed under reduced pressure at 150°C to obtain an epoxy resin. The above-described operation was performed plural times until 10 kg of epoxy resin was obtained. Then, a predetermined amount of the obtained epoxy resin was filled into an 18-L pail can, warmed at 150°C for 6 hours, and then crystallized at 20°C for 60 days to obtain a polycrystalline body of biphenol type diglycidyl ether of Example 3 (production method (3)). With regard to the epoxy resin filled into the pail can, crystallization days before the polycrystalline body was obtained by the above-described method were evaluated. Each measurement and evaluation described above were performed using the polycrystalline body.

These results are set forth in Table 1.

[Example 4]

An epoxy resin was obtained by an operation similar to that in the production example described above. Then, in an autoclave equipped with a thermometer, a stirring apparatus, and a cooling pipe, and having an inner capacity of 10 L, 2500 g of the obtained epoxy resin, 3000 g of methyl isobutyl ketone, and 750 g of dimethylsulfoxide were loaded, and homogeneously dissolved by increasing a reaction temperature to 65°C, followed by adding 123 g of 8% by mass potassium hydroxide/isopropanol solution to perform reaction for 2 hours. Then, 750 g of methyl isobutyl ketone was

added, and water washing was performed four times by using 1000 g of water. Then, methyl isobutyl ketone was completely removed under reduced pressure at 150°C to obtain an epoxy resin. The above-described operation was performed plural times until 10 kg of epoxy resin was obtained. Then, a predetermined amount of the obtained epoxy resin was filled into an 18-L pail can, warmed at 150°C for 6 hours, and then crystallized at 20°C for 60 days to obtain a polycrystalline body of biphenol type diglycidyl ether of Example 4 (production method (3)). With regard to the epoxy resin filled into the pail can, crystallization days before the polycrystalline body was obtained by the above-described method were evaluated. Each measurement and evaluation described above were performed using the polycrystalline body.

These results are set forth in Table 1.

[Comparative Example 1]

An epoxy resin was obtained by an operation similar to that in the production example described above. Then, in an autoclave equipped with a thermometer, a stirring apparatus, and a cooling pipe, and having an inner capacity of 10 L, 2500 g of the obtained epoxy resin, 3000 g of methyl isobutyl ketone, and 750 g of dimethylsulfoxide were loaded, and homogeneously dissolved by increasing a reaction temperature to 65°C, followed by adding 246 g of 8% by mass potassium hydroxide/isopropanol solution to perform reaction for 1 hour. Then, 750 g of methyl isobutyl ketone was added, and water washing was performed four times by using 1000 g of water. Then, methyl isobutyl ketone was completely removed under reduced pressure at 150°C to obtain an epoxy resin composition. The above-described operation was performed plural times until 10 kg of epoxy resin was obtained. Then, a predetermined amount of the obtained epoxy resin was filled into an 18-L pail can, warmed at 150°C for 6 hours, and then crystallized at 20°C for 60 days to obtain a polycrystalline body of biphenol type diglycidyl ether of Example 4. With regard to the epoxy resin filled into the pail can, crystallization days before the polycrystalline body was obtained by the above-described method were evaluated. Each measurement and evaluation described above were performed using the polycrystalline body.

These results are set forth in Table 1.

[Comparative Example 2]

The measurement of an epoxy equivalent (g/eq) and XRD, the calculation of a melt viscosity (poise) at 150°C and the contents (%) of epoxy resins (1-1) and (2-1), and the measurement of a melting point (°C) were performed using the resultant obtained by carrying out a step similar to that in Comparative Example 1 except that the condition of crystallization treatment in an 18-L pail can was changed from "at 20°C for 60 days" to "at 5°C for 60 days". The resultant was not a crystalline body, and an amorphous body (viscous liquid) having a low softening point was obtained. Therefore, grinding treatment was not performed.

These results are set forth in Table 1.

[Table 1]

Table 1

| Peak number Diffraction angle 2θ (°) | Diffraction angle 2θ | Example 1 | Example 2 | Example 3 | Example 4 | Comparative Example 1 | Comparative Example 2 |
|---|---|---|---|---|---|---|---|
| Crystallization temperature (°C) | | 20 | 20 | 20 | 20 | 20 | 5 |

(continued)

| Peak number Diffraction angle 2θ (°) | Diffraction angle 2θ | Example 1 | Example 2 | Example 3 | Example 4 | Comparative Example 1 | Comparative Example 2 |
|---|---|---|---|---|---|---|---|
| Peak 1 | Diffraction angle 2θ(°) | 9.139 | 9.239 | 9.139 | 9.252 | 9.090 | No peak was detected |
| 8.9~9.3 | Crystallite size (Å) | 503 | 602 | 491 | 418 | 335 | |
| Peak 2 | Diffraction angle 2θ(°) | 15.406 | 15.431 | 15.348 | 15.437 | 15.273 | |
| 15.1~15.5 | Crystallite size (Å) | 432 | 439 | 410 | 312 | 288 | |
| Peak 3 | Diffraction angle 2θ(°) | 19.655 | 19.721 | 19.647 | 19.710 | 19.531 | |
| 19.4~19.8 | Crystallite size (Å) | 392 | 434 | 380 | 301 | 250 | |
| Peak 4 | Diffraction angle 2θ(°) | 25.145 | 25.198 | 25.127 | 25.189 | 25.012 | |
| 24.9~25.3 | Crystallite size (Å) | 338 | 387 | 324 | 273 | 249 | |
| Epoxy equivalent (g/eq) | | 177 | 185 | 197 | 206 | 213 | 213 |
| Melting point (°C) | | 115 | 106 | 104 | 96, 104 | 76, 96 | No peak was detected |
| ICI viscosity at 150°C (Poise) | | 0.01 | 0.05 | 0.08 | 0.15 | 0.25 | 0.25 |
| Compound (1-1) (% by weight) | | 99.9 | 90.0 | 81.7 | 75.3 | 77.4 | 77.4 |
| Compound (2-1) (% by weight) | | 0 | 0 | 0.40 | 0.83 | 0.92 | 0.92 |
| Time (days) required for crystallization | | 1.5 | 2.0 | 17 | 28 | 54 | Not crystallized |
| Grindability | | A | A | A | A | B | B |
| Average particle diameter(μm) | | 17 | 359 | 465 | 2487 | - | - |
| D10(μm) | | 7 | 129 | 164 | 554 | - | - |
| D50(μm) | | 15 | 284 | 378 | 2257 | - | - |
| D90(μm) | | 28 | 691 | 825 | 3983 | - | - |

[Evaluation of Results]

Based on Examples 1 to 4, step time can be shortened because the polycrystalline body of the biphenol type diglycidyl ether according to the present embodiment is crystallized in a short period, and a cleaning load at the time of the occurrence of adhesion can be reduced because adhesion does not occur in an apparatus in grinding.

Moreover, the polycrystalline body of the biphenol type diglycidyl ether according to the present embodiment has a low melt viscosity, is therefore excellent in kneading properties in production of a sealing material and the filling properties of an inorganic filler, and can provide a semiconductor sealing material with high fluidity, in which wire flow in production of a semiconductor is prevented.

As a result, the polycrystalline body of the biphenol type diglycidyl ether according to the present embodiment is excellent in grindability and fluidity, and can improve productivity in production of an epoxy resin, a semiconductor sealing material, and the like. Moreover, the polycrystalline body of the biphenol type diglycidyl ether according to the present

embodiment can improve production efficiency in production of the polycrystalline body in itself, and can reduce a work load.

## Claims

1. A polycrystalline body of biphenol type diglycidyl ether, comprising an epoxy compound represented by the following Formula (1), and having a crystallite size of 355 Å or more and 100000 Å or less, calculated from a peak having a diffraction angle (2θ) of 8.9 to 9.3 deg in a powder X-ray diffraction pattern measured with a CuKα ray,

[Chem 1]

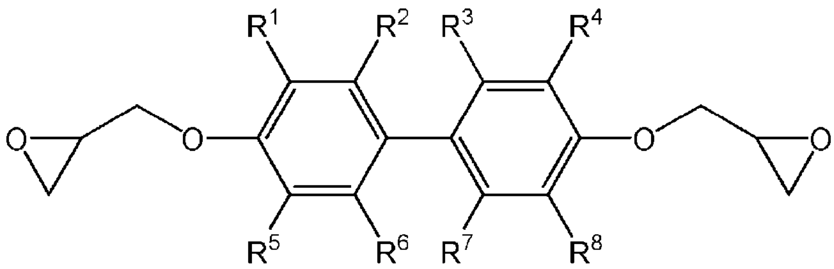

(1)

(in Formula (1) described above, each of $R^1$ to $R^8$ is independently a hydrogen atom, an alkyl group that may have a substituent and has 1 to 12 carbon atoms, an alkoxy group that may have a substituent and has 1 to 12 carbon atoms, an aryl group that may have a substituent and has 6 to 12 carbon atoms, an alkenyl group that may have a substituent and has 2 to 12 carbon atoms, or an alkynyl group that may have a substituent and has 2 to 12 carbon atoms).

2. The polycrystalline body of the biphenol type diglycidyl ether according to claim 1, wherein the polycrystalline body has an average particle diameter of 0.5 to 10000 μm.

3. The polycrystalline body of the biphenol type diglycidyl ether according to claim 1 or 2, wherein the polycrystalline body is a ground product.

4. The polycrystalline body of the biphenol type diglycidyl ether according to claim 1 or 2, wherein the polycrystalline body has a melting point of 80°C or more.

5. The polycrystalline body of the biphenol type diglycidyl ether according to claim 1 or 2, wherein the polycrystalline body has a crystallite size of 294 Å or more and 100000 Å or less, calculated from a peak having a diffraction angle (2θ) of 15.1 to 15.5 deg in the powder X-ray diffraction pattern measured with the CuKα ray.

6. The polycrystalline body of the biphenol type diglycidyl ether according to claim 1 or 2, wherein the polycrystalline body further has diffraction peaks at diffraction angles (2θ) of 15.1 to 15.5 deg, 19.4 to 19.8 deg, and 24.9 to 25.3 deg in the powder X-ray diffraction pattern measured with the CuKα ray.

7. The polycrystalline body of the biphenol type diglycidyl ether according to claim 1 or 2, wherein the polycrystalline body further comprises an epoxy compound represented by the following Formula (2):

[Chem 2]

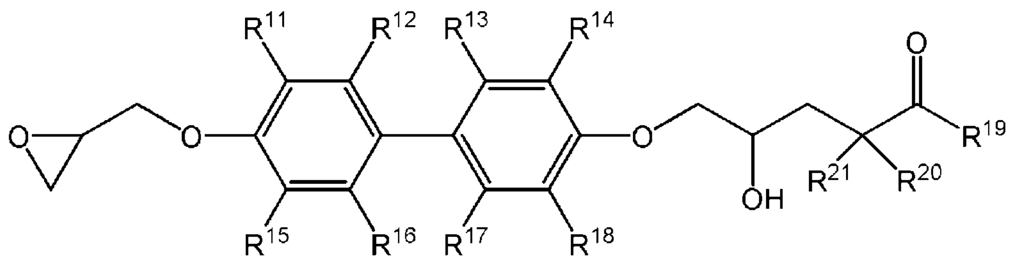

(2)

(in Formula (2), each of $R^{11}$ to $R^{18}$ is independently a hydrogen atom, an alkyl group that may have a substituent and has 1 to 12 carbon atoms, an alkoxy group that may have a substituent and has 1 to 12 carbon atoms, an aryl group that may have a substituent and has 6 to 12 carbon atoms, an alkenyl group that may have a substituent and has 2 to 12 carbon atoms, or an alkynyl group that may have a substituent and has 2 to 12 carbon atoms; each of $R^{19}$ to $R^{21}$ is independently a hydrogen atom, an alkyl group that may have a substituent and has 1 to 12 carbon atoms, or an aryl group that may have a substituent and may have a substituent having 6 to 12 carbon atoms; and $R^{19}$ and $R^{20}$ may be bound to each other to form a cyclic structure having 1 to 12 carbon atoms).

**8.** A resin composition comprising: the polycrystalline body of the biphenol type diglycidyl ether according to claim 1 or 2; and a curing agent, wherein the resin composition comprises 0.01 to 1000 parts by mass of the curing agent with respect to 100 parts by mass of a total epoxy resin component as a solid content.

**9.** The resin composition according to claim 8, wherein the curing agent is at least one selected from a group consisting of phenolic curing agents, amine-based curing agents, acid anhydride-based curing agents, and amide-based curing agents.

**10.** A cured product of the resin composition according to claim 8.

**11.** An electric and electronic component comprising a resin composition comprising the polycrystalline body of the biphenol type diglycidyl ether according to claim 1 or 2.

**12.** An electric and electronic component comprising a cured product of a resin composition comprising the polycrystalline body of the biphenol type diglycidyl ether according to claim 1 or 2.

## INTERNATIONAL SEARCH REPORT

International application No.
**PCT/JP2024/000435**

**A. CLASSIFICATION OF SUBJECT MATTER**

***C07D 303/27***(2006.01)i; ***C08G 59/06***(2006.01)i
FI: C07D303/27; C08G59/06

According to International Patent Classification (IPC) or to both national classification and IPC

**B. FIELDS SEARCHED**

Minimum documentation searched (classification system followed by classification symbols)

C07D303/27; C08G59/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 07-258384 A (NITTO DENKO CORPORATION) 09 October 1995 (1995-10-09) claim 1, paragraphs [0010]-[0012], [0041] | 1-6, 8-12 |
| A | | 7 |
| X | JP 08-239556 A (NITTO DENKO CORPORATION) 17 September 1996 (1996-09-17) claim 3, paragraphs [0009], [0025] (example 1) | 1-6, 8-12 |
| A | | 7 |
| X | JP 08-239557 A (NITTO DENKO CORPORATION) 17 September 1996 (1996-09-17) claim 3, paragraphs [0009], [0025] (example 1) | 1-6, 8-12 |
| A | | 7 |
| X | JP 2005-162940 A (TOTO KASEI CO., LTD.) 23 June 2005 (2005-06-23) paragraph [0026] | 1-6, 8-12 |
| A | | 7 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 March 2024** | **19 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/000435**

**C. DOCUMENTS CONSIDERED TO BE RELEVANT**

| Category* | Citation of document, with indication, where appropriate, of the relevant passages | Relevant to claim No. |
|---|---|---|
| X<br>A | JP 11-172075 A (SUMITOMO BAKELITE CO., LTD.) 29 June 1999 (1999-06-29)<br>claim 1, paragraphs [0010], [0012] (example 1), paragraphs [0025]-[0029] (example 4) | 1-6, 8-12<br>7 |

Form PCT/ISA/210 (second sheet) (July 2022)

**INTERNATIONAL SEARCH REPORT**
**Information on patent family members**

International application No.
**PCT/JP2024/000435**

| Patent document cited in search report | Publication date (day/month/year) | Patent family member(s) | Publication date (day/month/year) |
| --- | --- | --- | --- |
| JP 07-258384 A | 09 October 1995 | (Family: none) | |
| JP 08-239556 A | 17 September 1996 | (Family: none) | |
| JP 08-239557 A | 17 September 1996 | (Family: none) | |
| JP 2005-162940 A | 23 June 2005 | (Family: none) | |
| JP 11-172075 A | 29 June 1999 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 58039677 A
- JP 2004315832 A